# EUROPEAN PATENT APPLICATION

(11) **EP 1 709 971 A1**
(43) Date of publication of application: **11.10.2006**
(21) Application number: 05007552.2
(22) Date of filing: 06.04.2005
(51) Int. Cl.: A61K 39/285, C07K 7/06, C07K 16/08, C12N 15/63, C12N 5/10, A61P 31/20

(54) **Peptide antigens useful for the prophylaxis, treatment and diagnosis of poxvirus infections**

(71) Applicant: Ganymed Pharmaceuticals AG, 55131 Mainz (DE)
(72) Inventor: Wölfel, Thomas, 55128 Mainz (DE); Sahin, Ugur, 55116 Mainz (DE); Graf, Claudine, 67728 Münchweiler (DE); Britten, Cedrik, 55131 Mainz (DE); Meyer, Ralf Georg, 55116 Mainz (DE); Lennerz, Volker, 55128 Mainz (DE)
(74) Representative: Schnappauf, Georg

(57) **Abstract**

The present invention generally relates to the prophylaxis, treatment and diagnosis of infections caused by poxviruses, in particular viruses of the genus Orthopoxvirus. In one specific embodiment, the present invention provides peptides which are recognized as peptide antigens by the immune system, in particular by CD8-positive cytotoxic T lymphocytes (CTL), and thus elicit an immune response against poxviruses.

SEQUENCE LISTING

<110>  Ganymed Pharmaceuticals AG

<120>  PEPTIDE ANTIGENS USEFUL FOR THE PROPHYLAXIS, TREATMENT AND DIAGNOSIS OF
POXVIRUS INFECTIONS

<130>  342-24

<160>  3

<170>  PatentIn version 3.1

<210>  1
<211>  9
<212>  PRT
<213>  Artificial

<220>
<223>  Peptide

<400>  1

Ser Ile Ile Asp Leu Ile Asp Glu Tyr
1               5


<210>  2
<211>  9
<212>  PRT
<213>  Artificial

<220>
<223>  Peptide

<400>  2

Phe Gln Gln Lys Val Leu Gln Glu Tyr
1               5


<210>  3
<211>  9
<212>  PRT
<213>  Artificial

<220>
<223>  Peptide

<400>  3

Asp Glu Val Lys Gly Leu Thr Val Phe
1               5

## Description

### FIELD OF THE INVENTION

The present invention generally relates to the prophylaxis, treatment and diagnosis of infections caused by poxviruses, in particular viruses of the genus Orthopoxvirus. In one specific embodiment, the present invention provides peptides which are recognized as peptide antigens by the immune system, in particular by CD8-positive cytotoxic T lymphocytes (CTL), and thus elicit an immune response against poxviruses.

### BACKGROUND OF THE INVENTION

Viruses in the family Poxviridae are characterized by a large linear double-stranded DNA genome (130-300 kb) packaged in a relatively large virion (350 X 270 nm), and a cytoplasmic site of replication (reviewed by Moss, 1996, In "Fields Virology", D. M. Knipe et al. (eds.), 3rd edition, pp 2637-2702, Lippincott-Raven, Philadelphia).

Variola virus, the causative agent of smallpox, is a member of the Orthopoxvirus genus, which also includes monkeypox, cowpox, and vaccinia viruses. The disease caused by variola major strains is characterized by a low infectious dose (10-100 virions), long incubation period (averaging 12 days), fever, constitutional symptoms, rash progressing to a pustular stage, death in up to 30% of those affected, and facial scarring in survivors. The disease is spread person-to-person via the respiratory route by contact (droplets) and, possibly, by aerosol.

Smallpox was one of the most important causes of morbidity and mortality worldwide throughout the first half of the 20century. However, in part because of the lack of animal reservoir for the virus, the systematic use of a vaccine (live, attenuated vaccinia virus) was highly effective in fighting this disease. Indeed, between 1967-1977, a global program of smallpox eradication resulted in the elimination of the natural disease (Fenner et al., WHO, Geneva, p. 1460, 1988). Because of the absence of smallpox and the risk of vaccine-associated adverse events, routine vaccination of children, hospital personnel, and military personnel has ceased, and only persons working with vaccinia and related viruses in the laboratory are currently immunized. Thus, a substantial portion of the world's population has no immunity to smallpox. The remaining population has little residual immunity, as vaccine immunity lasts only 5 years after primary vaccination and less than 20 years after revaccination. The eradication of smallpox and the cessation of vaccination have, thus, created vulnerability in the population to covert attack or biowarfare employing variola virus. Should such an event occur, epidemic spread would be unchecked by an immune barrier in the population (Anon. (Editorial), Lancet 353:1539, 1999; Henderson, 1999, Science 283:1279-1282; Henderson et al., 1999, J.A.M.A. 281:2127-2137).

Because of the uncertainties surrounding smallpox eradication, vaccine was stockpiled for emergency use. In the United States, for example, 155,000 vaccine vials (nominally 15.5 million doses) produced by Wyeth Laboratories were originally stockpiled under the control of the Centers for Disease Control and Prevention (CDC), Atlanta, Georgia, U.S. At a meeting of the National Vaccines Advisory Committee in January 1999, the CDC reported on the status of the national smallpox vaccine repository. At that time, of the 15.5 million doses held by Wyeth, 3.4 million doses had failed quality control testing and 10.3 million were beyond the expiration date specified by the last control test for extended dating, leaving 1.7 million doses that met release specifications (LeDuc, Presentation to the National Vaccines Advisory Committee, Washington D.C., Jan. 11-12, 1999). In addition to the limited supply, the vaccine is packaged in 100 dose vials, which restricts distribution and increases the likelihood of wastage during an emergency.

In addition to the U.S. stockpile, there is a supply of vaccine (Lister, Elstree strain) stored at the National Institute of Public Health, Bilthoven, Netherlands, and certain other countries have supplies of smallpox vaccine, which at the time of eradication may have included up to 300 million doses. However, similar problems of stability in storage have reduced this supply to less than 50 million doses (Henderson, 1999, Science 283:1279-1282).

Vaccinia virus, the prototypical member of the poxvirus family which served as an effective vaccine in the global eradication of smallpox has been engineered as a vaccine vector against a myriad of infectious diseases and malignancies (Carroll, M. W. et al., 1998, J. Natl. Cancer Inst. 90, 1881-1887, Giavedoni, L. et al., 1991, Proc. Natl. Acad. Sci. USA 88, 8011-8015, Pastoret, P. P. and Brochier B., 1996, Epidemiol. Infect. 116, 235-240). Many recombinant Vaccinia virus vectors have been shown to elicit potent and protective humoral and cell-mediated immune responses (Belyakov, I. M. et al., 1999, Proc. Natl. Acad. Sci. USA 96, 4512-4517, Gherardi, M. M. et al., 1999, J. Immunol. 162, 6724-6733, Marshall, J. L. et al., 2000, J. Clin. Oncol. 18, 3964-3973).

Assembly of Vaccinia virus virions begins with condensation of dense granular material into membrane-wrapped particles called intracellular mature virions (IMV). Recent findings indicate the IMV are wrapped by a single membrane (Hollingshead et al., 1999, J. Virol. 73, 1503-1517) rather than a double membrane as previously reported. IMV are then enveloped in two additional membranes derived from the trans Golgi to form multiple membrane-wrapped particles called intracellular enveloped virions (IEV) (Schmelz et al., 1994, J. Virol. 68, 130-147). IEV are moved, possibly by actin polymerization (Cudmore et al., 1995, Nature 378, 636-638), to the cell periphery, where the outermost membrane fuses with the cell plasma membrane, exposing a cell-associated enveloped virion (CEV) (Blasco and Moss, 1991, J. Virol. 65, 5910-5920). CEV are released from the cell as extracellular enveloped virions (EEV), which play a role in long-range spread of the virus (Payne, 1980, J. Gen. Virol. 50, 89-100). IMV released from disrupted cells and EEV are both infectious forms of Vaccinia virus.

Although Vaccinia virus has not been directly associated with any specific disease, serious complications such as dermatologic and central nervous system disorders have occurred, primarily in immunosuppressed populations (Arita, I. and Fenner F., 1985, Complications of smallpox vaccination, p. 49-60. In G. V. Quinnan, Jr. (ed.), Vaccinia viruses as vectors for vaccine antigens: proceedings of the Workshop on Vaccinia Viruses as Vectors for Vaccine Antigens. Elsevier, New York, N.Y., Baxby, D., 1991, Lancet 337, 913, Cooney, E. L. et al., 1991, Lancet 337, 567-572, Redfield, R. R. et al., 1987, N. Engl. J. Med. 316, 673-676).

The primary therapeutic tool for the control and eradication of infection with Vaccinia virus include a live virus vaccine to prevent disease, and a vaccinia immune globulin (VIG) to treat disseminated infections.

The existing VIG product is derived from human donors who have been vaccinated with the smallpox vaccine, Vaccinia virus. As with all human products, the existing VIG must be tested exhaustively for blood borne human pathogens such as human immunodeficiency virus and hepatitis B. Therefore, the existing VIG suffers from several drawbacks including the necessity for using human volunteers, i.e. the use of a live virus as an immunogen which could cause infectious lesions that scar in healthy individuals and severe disseminated life-threatening infection in immunocompromised individuals. And, despite continuous screening of the donor population to assure consistency which is very expensive, product lots can vary significantly between batches and geographic regions.

Increased immunosuppression as a result of human immunodeficiency virus infection, cancer treatments, and organ transplantation, in addition to the possible vaccination of the general public due to the emerging threat of smallpox bioterrorism, underscores the need for the development of safer yet efficacious Vaccinia virus vaccine which is precisely defined, and which does not rely on human donors. Vaccinia virus has been effectively utilized as a live vaccine against smallpox as well as a vector for vaccine development and immunotherapy. Increasingly there is a need for a new generation of highly attenuated and efficacious Vaccinia virus vaccines, especially in light of the AIDS pandemic and the threat of global bioterrorism.

The development of recombinant vaccines for the prevention or therapy of infectious diseases depends essentially on molecularly defined antigens. A prerequisite for the development of immunotherapeutic procedures for the prophylaxis and treatment of viral infections is the identification of immunogenic antigens. Such antigens can be employed under certain conditions as a vaccine for the induction of T cells and/or antibodies with the aim that these T cells and/or antibodies prevent, ameliorate or eradicate a viral infection.

Even though Vaccinia is one of the most extensively studied pathogens, molecular specificities of antiviral immunity have not been studied comprehensively. In particular, substantial information about relevant Orthopoxvirus antigens and epitope specificities of virus specific CTL responses is still lacking, only a few MHC presented epitopes having been identified so far (Terajima, M. et al., 2003, J. Exp. Med. 197, 927-932, Drexler, I. et al., 2003, Proc. Natl. Acad. Sci. U.S.A 100, 217-222, Snyder, J. T. et al., 2004, J. Virol. 78, 7052-7060).

The present invention provides novel sero- and T cell recognized antigens for poxviruses. These antigens used alone, combined for synergistic effects, or together with attenuated vaccines to reduce their side-effects provide the basis for new and safe approaches in the prophylaxis, treatment and diagnosis of infections caused by poxviruses, in particular of the genus Orthopoxvirus. Furthermore, the antigens described herein are of value for virus specific immune monitoring, including studying T cell kinetics after vaccination and evaluating poxvirus specific immune responses in mammals, specifically humans.

### SUMMARY OF THE INVENTION

The present invention generally provides compositions and methods for the diagnosis, prophylaxis and therapy of infections caused by poxviruses, in particular viruses of the genus Orthopoxvirus.

In one aspect, the present invention provides peptides comprising an immunogenic or antigenic portion of an Orthopoxvirus antigen, or a variant thereof. Specifically, the present invention provides a peptide comprising an amino acid sequence as set forth in any one of SEQ ID NO: 1 to 3, or a variant thereof, having substantially identical biological properties, with the proviso that the peptide is not a naturally occurring virus protein, in particular of the genus Orthopoxvirus. In one embodiment, the peptide has an amino acid sequence as set forth in any one of SEQ ID NO: 1 to 3. Preferably, the peptides and variants according to the present invention are capable of inducing an antigenic and/or immunogenic reaction against Orthopoxvirus infection when administered to patients. In a preferred embodiment the peptides and variants bind a to a HLA molecule, preferably HLA-B, which HLA molecule may optionally be present on an antigen presenting cell. In a further preferred embodiment the peptides and variants according to the present invention are recognized by cytotoxic T lymphocytes and/or activate and/or induce cytotoxic T lymphocytes. In a further preferred embodiment the peptides and variants according to the present invention are recognized by cytotoxic T lymphocytes in an HLA-B-restricted manner and/or activate and/or induce cytotoxic T lymphocytes in an HLA-B-restricted manner. In a further embodiment, the peptides and variants according to the present invention are capable of binding to HLA-B.

The present invention also provides nucleic acids that encode a peptide as described above, expression vectors comprising such nucleic acids and host cells transformed or transfected with such nucleic acids and/or expression vectors.

The host cells may also comprise a nucleic acid coding for a HLA molecule. In one embodiment, the host cell endogenously expresses the HLA molecule. In a further embodiment, the host cell expresses the recombinant HLA molecule and/or the nucleic acid of the invention. Preferably, the host cell is non-proliferative. In a preferred embodiment, the host cell is an antigen-presenting cell, in particular a dendritic cell, a monocyte or a macrophage.

In a further aspect, the invention relates to an agent that binds to a peptide or variant as described above. In certain embodiments of this aspect, the binding agent is an antibody, a T cell or a T cell receptor which specifically binds to a peptide or variant as described above or to a complex of said peptide or variant and a HLA molecule, preferably HLA-B. In further embodiments, the antibody is a chimeric, a humanized antibody or an antibody produced by combinatory techniques or is a fragment of an antibody.

Furthermore, the invention relates to an antibody which binds selectively to a complex of (i) a peptide or variant as described above and (ii) an HLA molecule to which said peptide or variant binds, with said antibody not binding to (i) or (ii) alone. An antibody of the invention may be a monoclonal antibody. In further embodiments, the antibody is a chimeric or humanized antibody or a fragment of a natural antibody.

The invention furthermore provides a conjugate between an agent of the invention which binds to a peptide or variant according to the invention or an antibody of the invention and a therapeutic or diagnostic agent. In one embodiment, the therapeutic or diagnostic agent is a toxin.

In a further aspect, the invention provides a pharmaceutical composition comprising an agent which recognizes a peptide comprising an amino acid sequence as set forth in any one of SEQ ID NO: 1 to 3, or a variant thereof having substantially identical biological properties, and which is preferably selective for cells presenting said peptide or variant. In particular embodiments, said agent may cause induction of cell death, reduction in cell growth and/or damage to the cell membrane or secretion of cytokines. In one embodiment, the agent is an antibody which binds selectively to the peptide or variant. In one embodiment of this aspect of the invention, the peptide or variant serves as a label for recruiting effector mechanisms. In a preferred embodiment, the agent is a cytotoxic T lymphocyte which recognizes the peptide or variant on an HLA molecule and lyses the cell labeled in this way. In a further embodiment, the agent is an antibody which binds selectively to the peptide or variant and thus recruits natural or artificial effector mechanisms to a cell. In a further embodiment, the agent is a T helper lymphocyte which enhances effector functions of other cells specifically recognizing said peptide or variant.

The invention furthermore provides a pharmaceutical composition which comprises an agent which, when administered, selectively increases the amount of complexes between an HLA molecule and a peptide comprising an amino acid sequence as set forth in any one of SEQ ID NO: 1 to 3, or a variant thereof having substantially identical biological properties. In one embodiment, the agent comprises one or more components selected from the group consisting of (i) a peptide comprising an amino acid sequence as set forth in any one of SEQ ID NO: 1 to 3, or a variant thereof having substantially identical biological properties, (ii) a nucleic acid which codes for said peptide or variant, (iii) a host cell which expresses said peptide or variant, and (iv) isolated complexes between said peptide or variant and an HLA molecule. In one embodiment, the agent comprises two or more agents which each selectively increase the amount of complexes between HLA molecules and different peptides comprising an amino acid sequence as set forth in any one of SEQ ID NO: 1 to 3, or variants thereof.

The invention furthermore relates to a pharmaceutical composition which comprises one or more components selected from the group consisting of (i) a peptide comprising an amino acid sequence as set forth in any one of SEQ ID NO: 1 to 3, or a variant thereof having substantially identical biological properties, (ii) a nucleic acid which codes for said peptide or variant, (iii) a host cell which expresses and/or presents said peptide or variant, (iv) an antibody which binds to said peptide or variant, (v) a T cell or T cell receptor which binds to said peptide or variant, (vi) isolated complexes between said peptide or variant and an HLA molecule.

A nucleic acid coding for the peptide or variant may be present in the pharmaceutical composition of the present invention in an expression vector and functionally linked to a promoter.

A host cell present in the pharmaceutical composition of the invention may secrete the peptide or variant, express it on the surface or may additionally express an HLA molecule which binds to the peptide or variant. A host cell that presents the peptide or variant may also be a cell which has been loaded with said peptide or variant. In one embodiment, the host cell expresses the HLA molecule endogenously. In a further embodiment, the host cell expresses the HLA molecule and/or the peptide or variant in a recombinant manner. The host cell is preferably non-proliferative. In a preferred embodiment, the host cell is an antigen-presenting cell, in particular a dendritic cell, a monocyte or a macrophage.

An antibody present in the pharmaceutical composition of the invention may be a monoclonal antibody. In further embodiments, the antibody is a chimeric or humanized antibody, a fragment of a natural antibody or a synthetic antibody, all of which may be produced by combinatory techniques. The antibody may be coupled to a therapeutically or diagnostically useful agent.

In further embodiments, a peptide or variant provided by a pharmaceutical composition of the invention either directly or via expression of a nucleic acid binds to HLA molecules on the surface of cells, said binding preferably causing a cytolytic response and/or inducing cytokine release.

The pharmaceutical composition of the invention may comprise a pharmaceutically compatible carrier and/or an adjuvant. The adjuvant may be selected from saponin, GM-CSF, CpG nucleotides, RNA, a cytokine or a chemokine. A pharmaceutical composition of the invention is preferably used for the prophylaxis and/or treatment of Orthopoxvirus infection.

In a particularly preferred embodiment the pharmaceutical composition is in the form of a vaccine.

In a particularly preferred embodiment the pharmaceutical composition of the present invention comprises T cells or antigen presenting cells that have been incubated with a peptide or variant, nucleic acid or expression vector as described above. Within related aspects, vaccines are provided that comprise: (a) an antigen presenting cell that expresses a peptide as described above and (b) an immunostimulant.

In a further preferred embodiment of the pharmaceutical compositions of the present invention, the peptide or variant is not a naturally occurring virus protein and the nucleic acid or expression vector does not encode a naturally occurring virus protein. In a further preferred embodiment, the peptide has an amino acid sequence as set forth in any one of SEQ ID NO: 1 to 3 and the nucleic acid or expression vector encodes said peptide.

In yet another aspect, methods are provided for stimulating an immune response in a patient, e.g., for inducing protective immunity in a patient, comprising administering to a patient an effective amount of the above pharmaceutical compositions or vaccines. Preferably, the stimulation of an immune response in a patient comprises an activation and/or expansion of T cells, in particular CD8-positive cytotoxic T lymphocytes in said patient.

In a further aspect the present invention relates to a kit for diagnosis of Orthopoxvirus infection in a biological sample and/or determining immune reactivity of a biological sample against Orthopoxvirus, which comprises a peptide comprising an amino acid sequence as set forth in any one of SEQ ID NO: 1 to 3, or a variant thereof having substantially identical biological properties. In a preferred embodiment of this aspect the peptide or variant is not a naturally occurring virus protein. In a further preferred embodiment, the peptide or variant has an amino acid sequence as set forth in any one of SEQ ID NO: 1 to 3. The diagnostic kit is useful for determination of T cell immune reactivity against Orthopoxviruses and/or determination of total antibodies, antibody IgG and/or antibodies IgM against Orthopoxviruses in a biological sample. In a preferred embodiment the peptide or variant is pre-coated on the surface of a suitable support.

The aforementioned peptides and variants are suitable both for the in-vivo induction of T lymphocytes in the patient and for the in-vitro induction and expansion of appropriately reactive patient's own or patient-foreign T lymphocytes.

Accordingly, in this aspect, the present invention relates to the use of a peptide comprising an amino acid sequence as set forth in any one of SEQ ID NO: 1 to 3, or a variant thereof having substantially identical biological properties, a nucleic acid encoding said peptide or variant, an expression vector comprising said nucleic acid, or any combination thereof, for the in-vivo- or in-vitro generation, activation and/or expansion of T cells, in particular CD8-positive cytotoxic T lymphocytes.

For an in-vivo induction and expansion of T lymphocytes in a patient various processes are possible, for example (a) the injection of the peptide and/or variant as pure peptide or together with adjuvants or with cytokines or in a suitable release systems such as, for example, liposomes, (b) the injection of one or more nucleic acids coding at least for the peptide or for its variants in "naked" or complexed form or in the form of viral or nonviral vectors or together with release systems such as cationic lipids or cationic polymers, (c) the loading of cells of autologous, allogenic, xenogenic or microbiological origin with the peptide or variants, so that as a result the peptide or its derivatives is presented on the respective cells, or (d) the transfection or infection of cells of autologous, allogenic, xenogenic or microbiological origin with a nucleic acid coding at least for the peptide or the variant (again either in "naked" or complexed form or in the form of viral or nonviral vectors).

In the case of an in-vitro induction and expansion, the T lymphocytes obtained in-vitro may then be administered to a patient by infusion or injection or like procedures.

In one embodiment of this aspect, the present invention relates to a method for inducing a cytotoxic T lymphocyte comprising the steps of: i) incubating an antigen-presenting cell with a peptide comprising an amino acid sequence as set forth in any one of SEQ ID NO: 1 to 3, or a variant thereof having substantially identical biological properties, or ii) expressing a peptide comprising an amino acid sequence as set forth in any one of SEQ ID NO: 1 to 3, or a variant thereof having substantially identical biological properties in an antigen-presenting cell; and iii) using the cell obtained in said step i) or said step ii) for stimulating a group of cells that contain a precursor cell of the cytotoxic T lymphocyte. In certain embodiments, the cytotoxic T lymphocyte and the antigen presenting cell are allogeneic (allorestricted) or syngeneic (self-restricted) with respect to the HLA molecule expressed by the antigen presenting cell. In a further embodiment, the HLA molecule expressed by the antigen-presenting cell is HLA-B, more preferably HLA-B*1501 or HLA-B*1801.

In a preferred embodiment of the aformentioned aspects of the present invention, the peptide or variant is not a naturally occurring virus protein and the nucleic or expression vector does not encode a naturally occurring virus protein. In a further preferred embodiment, the peptide has an amino acid sequence as set forth in any one of SEQ ID NO: 1 to 3 and the nucleic or expression vector encodes said peptide.

The cytotoxic T lymphocytes obtainable by the above-described methods may be administered to a patient in a prophylaxis and/or treatment effective amount for prophylaxis and/or treatment of Orthopoxvirus infection in a patient.

In a further aspect, the present invention provides a method of determining the T cell immune reactivity of a mammal against Orthopoxvirus comprising contacting a sample from said mammal with a peptide comprising an amino acid sequence as set forth in any one of SEQ ID NO: 1 to 3, or a variant thereof having substantially identical biological properties and/or a complex between said peptide or variant and a HLA molecule and/or a complex between antigen presenting cells and said peptide or variant and determining the reactivity of T cells contained in said sample. In a preferred embodiment, the peptide is not a naturally occurring virus protein. In a further preferred embodiment, the peptide has an amino acid sequence as set forth in any one of SEQ ID NO: 1 to 3. In a further preferred embodiment the method is useful for determining the protection in said mammal against Orthopoxvirus infection, wherein the protection is preferably provided by vaccination.

The invention furthermore relates to methods of preventing, treating or diagnosing Orthopoxvirus infection.

In one embodiment, the prevention or treatment comprises administering a pharmaceutical composition of the invention.

In one embodiment, the invention provides a method of treating Orthopoxvirus infection in a patient, which method comprises (i) removing a sample containing immunoreactive cells from said patient, (ii) contacting said sample with a host cell expressing a peptide comprising an amino acid sequence as set forth in any one of SEQ ID NO: 1 to 3, or a variant thereof having substantially identical biological properties, under conditions which favor production of cytolytic T cells against said peptide or variant, and (iii) introducing the cytolytic T cells into the patient in an amount suitable for lysing cells expressing said peptide or variant.

In one embodiment, the host cell endogenously expresses an HLA molecule. In a further embodiment, the host cell recombinantly expresses an HLA molecule and/or the peptide or variant. The host cell is preferably nonproliferative. In a preferred embodiment, the host cell is an antigen-presenting cell, in particular a dendritic cell, a monocyte or a macrophage.

In another embodiment methods for the prophylaxis and/or treatment of Orthopoxvirus infection are provided that comprise incubating antigen presenting cells with a peptide comprising an amino acid sequence as set forth in any one of SEQ ID NO: 1 to 3, or a variant thereof having substantially identical biological properties, a nucleic acid encoding said peptide or variant, an expression vector comprising said nucleic acid, or any combination thereof ex vivo to provide incubated antigen presenting cells and administering the incubated antigen presenting cells to the patient. Proliferated cells may, but need not, be cloned prior to administration to the patient. In certain embodiments, the antigen presenting cells are selected from the group consisting of dendritic cells, macrophages, monocytes, B-cells, and fibroblasts.

In a further aspect, the present invention provides methods for prophylaxis and/or treatment of Orthopoxvirus infection in a patient, which comprises administrating to the patient a prophylaxis and/or treatment effective amount of (i) a peptide comprising an amino acid sequence as set forth in any one of SEQ ID NO: 1 to 3, or a variant thereof having substantially identical biological properties, (ii) a nucleic acid which codes for said peptide or variant, (iii) a host cell which expresses and/or presents said peptide or variant, (iv) an antibody which binds to said peptide or variant, (v) a T cell or T cell receptor which binds to said peptide or variant, (vi) isolated complexes between said peptide or variant and an HLA molecule, or any combination thereof.

In a preferred embodiment of all above described aspects, the peptide or variant is not a naturally occurring virus protein and the nucleic or expression vector does not encode a naturally occurring virus protein. In a further preferred embodiment, the peptide has an amino acid sequence as set forth in any one of SEQ ID NO: 1 to 3 and the nucleic or expression vector encodes said peptide.

In further aspects of the present invention, methods are provided for detecting Orthopoxvirus infection in a patient.

In one aspect, the invention relates to a method of diagnosing Orthopoxvirus infection comprising detection of (i) a peptide comprising an amino acid sequence as set forth in any one of SEQ ID NO: 1 to 3, or a variant thereof having substantially identical biological properties, and/or (ii) detection of an antibody to the peptide or variant and/or (iii) detection of cytotoxic T lymphocytes which are specific for the peptide or variant in a biological sample isolated from a patient. In particular embodiments, detection comprises (i) contacting the biological sample with an agent which binds specifically to the peptide or variant, or to the antibody or to cytotoxic T lymphocytes specific for the peptide or variant, and (ii) detecting the formation of a complex between the agent and the peptide or variant, the antibody or the cytotoxic T lymphocytes. In a further embodiment, the biological sample isolated from the patient is compared to a comparable normal biological sample.

According to the invention, detection of a peptide or variant may be carried out using an antibody binding specifically to said peptide or variant.

In further embodiments, the peptide or variant to be detected is present in a complex with an HLA molecule, in particular an HLA-B molecule.

According to the invention, detection of an antibody may be carried out using a protein or peptide binding specifically to said antibody.

According to the invention, detection of cytotoxic T lymphocytes which are specific for complexes between an antigen and HLA molecules may be carried out using a cell presenting the complex between said antigen and an HLA molecule.

The antibody, the protein or peptide or the cell, which is used for detection or monitoring, is preferably labeled in a detectable manner. In particular embodiments, the detectable marker is a radioactive marker or an enzymic marker. T lymphocytes may additionally be detected by detecting their proliferation, their cytokine production, and their cytotoxic activity triggered by specific stimulation with the complex of HLA and antigen.

In one embodiment of this aspect, the present invention provides a method for diagnosis of Orthopoxvirus infection in a patient, comprising contacting a peptide comprising an amino acid sequence as set forth in any one of SEQ ID NO: 1 to 3, or a variant thereof having substantially identical biological properties, with a sample of a patient to be detected under conditions suitable for the interaction of antigen and antibody and/or antigen and T cells and detecting antibody and/or T cells bound to said peptide or variant. In certain embodiments, total antibodies against Orthopoxvirus, antibody IgG against Orthopoxvirus, and/or antibody IgM against Orthopoxvirus are detected in the biological sample.

These and other aspects of the present invention will become apparent upon reference to the following detailed description. All references disclosed herein are hereby incorporated by reference in their entirety as if each was incorporated individually.

### DETAILED DESCRIPTION OF THE PREFERRED EMBODIMENT

The subfamily Chordopoxviridae, of the family Poxviridae, comprises eight genera and a few unclassified species. Within the genus Orthopoxvirus the species Vaccinia virus, Variola virus, Monkeypox virus and Cowpox virus can cause human infections.

"Orthopoxvirus" as used herein includes Vaccinia virus, Variola virus, Monkeypox virus, Cowpox virus, Camelpox virus and Rabbitpox virus, Ectromelia virus. Preferably "Orthopoxvirus" as used herein refers to Vaccinia virus and Variola virus.

The terms "treatment of Orthopoxvirus infection" or "diagnosis of Orthopoxvirus infection" relates to the treatment or diagnosis of a viral infection caused by a virus of the genus Orthopoxvirus which is amenable to treatment or diagnosis by the treatment or diagnosis agents described herein. Preferably, a virus of the genus Orthopoxvirus which is amenable to treatment or diagnosis by the treatment or diagnosis agents described herein comprises a peptide or protein comprising an amino acid sequence as set forth in any one of SEQ ID NO: 1 to 3, or a variant thereof having substantially identical biological properties. In certain embodiments the infection is caused by a species selected from Vaccinia virus, Variola virus, Monkeypox virus, Cowpox virus, Camelpox virus and Rabbitpox virus, Ectromelia virus, preferably Vaccinia virus and Variola virus. A viral infection results from the exposure of a patient to the virus. In one embodiment exposure of a patient to the virus results from the use of a vaccine comprising or contaminated with the virus.

Here, to "recognize" means that a recognizing entity discerns a to-be-recognized target from others and may bind to the recognized target. In particular, in the present specification, recognition of a peptide by cytotoxic T lymphocytes means that a cytotoxic T lymphocyte binds to a peptide, preferably presented by an HLA molecule, via a T cell antigen receptor (hereinafter also abbreviated as "TCR").

As used herein, "binding" refers to a noncovalent association between two separate molecules such that a complex is formed. "Specific binding" means that a molecule reacts at a detectable level (within, for example, an ELISA) with another molecule, and does not react detectably with unrelated molecules under similar conditions. The ability to bind may be evaluated by, for example, determining a binding constant for the formation of the complex. The binding constant may be determined using methods well known in the art.

To "activate" means to further enhance or bring into action an entity or a state that has a given activity or effect. In particular, in the present specification, activation of a cytotoxic T lymphocyte means that a cytotoxic T lymphocyte generates, for example, IFN-gamma as a result of recognizing an antigen presented by an HLA molecule, or that a cytotoxic T lymphocyte demonstrates a cytotoxic activity against a target cell that has been recognized.

To "induce" means to generate a given activity or effect from an entity or a state that substantially lacks the activity or the effect. In particular, in the present specification, to induce a cytotoxic T lymphocyte means to cause a cytotoxic T lymphocyte that specifically recognizes a given antigen to differentiate and/or to proliferate in vitro or in vivo.

"Antigenicity" or "antigenic" relates to the ability of a substance to be effective as an antigen, i.e. to bind to an antibody molecule or to a T cell receptor (e.g. in an ELISA or other immunoassay).

"Immunogenicity" or "immunogenic" relates to the ability of an antigen or vaccine to stimulate or induce an immune response, e.g. the formation of immunologically competent cells and the synthesis of immunoglobulines (antibodies). Immunogenicity may generally be identified using well known techniques, such as those summarized in Paul, Fundamental Immunology, 3rd ed., 243-247 (Raven Press, 1993) and references cited therein. Such techniques include screening peptides for the ability to react with antigen-specific antibodies, antisera and/or T cell lines or clones.

An antigen is a macromolecule that is recognized by antibodies or immune cells and can trigger an immune response. Usually, an antigen is a protein or a polysaccharide, but it can be any type of molecule, even small molecules if coupled to a large carrier. Antigens are presented by a cell to its environment via a histocompatibility molecule. Depending on the antigen presented and the histocompatibility molecule used, several types of immune cells can leap into action. Exogenous antigens are antigens that have entered the body, e.g., by inhalation, ingestion, or injection. These antigens are taken up by endocytosis by the cell, and degraded into fragments. The fragments are then presented by class II histocompatibility molecules and attract phagocytic cells like macrophages and dendritic cells, as well as B lymphocytes (also called B cells) which can produce antibodies against this specific antigen. Endogenous antigens are antigens that have been generated within the cell, e.g., by a virus, and are degraded into fragments. The fragments are then presented by class I histocompatibility molecules and attract CD8+ T cells, most of which are cytotoxic and kill the infected cell, usually before any viruses are released from the infected cell. Cells display degraded proteins of all kinds this way, no matter if they are from a virus or just normal proteins of the cell. In order to keep the cytotoxic cells from killing cells just for presenting normal proteins, they run through a test cycle just after their production. Only the T cells that do not react to normal body protein fragments are allowed to enter the bloodstream. Antibodies also exist free floating through the bloodstream as part of the humoral immune system. Antibodies exist in clonal lines that are specific to only one antigen, e.g., a virus hull protein. In binding to such antigens, they can cause agglutination and precipitation of antibody-antigen products prime for phagocytosis by macrophages, block viral receptors and stimulate other immune responses such as the complement pathway.

An antigen presenting cell is a type of cell which provokes an immune response from T lymphocytes by binding foreign antigens to their surfaces and then interacting with the T cells. Certain preferred embodiments of the present invention use dendritic cells or progenitors thereof as antigen-presenting cells. Dendritic cells are highly potent APCs (Banchereau and Steinman, 1998, Nature 392, 245-251,) and have been shown to be effective as a physiological adjuvant for eliciting prophylactic or therapeutic immunity (see Timmerman and Levy, 1999, Ann. Rev. Med. 50, 507-529,). In general, dendritic cells may be identified based on their typical shape (stellate in situ, with marked cytoplasmic processes (dendrites) visible in vitro), their ability to take up, process and present antigens with high efficiency, and their ability to activate naive T cell responses. Dendritic cells may, of course, be engineered to express specific cell-surface receptors or ligands that are not commonly found on dendritic cells in vivo or ex vivo, and such modified dendritic cells are contemplated by the present invention.

Dendritic cells and progenitors may be obtained from peripheral blood, bone marrow, lymph nodes, spleen, skin, umbilical cord blood or any other suitable tissue or fluid. For example, dendritic cells may be differentiated ex vivo by adding a combination of cytokines such as GM-CSF, IL-4, IL-13 and/or TNF-alpha to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNF-alpha, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fc-gamma receptor and mannose receptor. The mature phenotype is typically characterized by a lower expression of these markers, but a high expression of cell surface molecules responsible for T cell activation such as class I and class II MHC, adhesion molecules (e.g., CD54 and CD11) and costimulatory molecules (e.g., CD40, CD80, CD86 and 4-1BB).

APCs may generally be transfected with a nucleic acid encoding a peptide such that the peptide, or an immunogenic portion thereof, is expressed on the cell surface. Such transfection may take place ex vivo, and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs in vivo. In vivo and ex vivo transfection of dendritic cells, for example, may generally be performed using any methods known in the art, such as those described in WO 97/24447, or the gene gun approach described by Mahvi et al., 1997, Immunology and Cell Biology 75, 456-460. Antigen loading of dendritic cells may be achieved by incubating dendritic cells or progenitor cells with an antigenic peptide, nucleic acid coding therefor (DNA or RNA); or with antigen-expressing recombinant bacterium or viruses (e.g., vaccinia, fowlpox, adenovirus or lentivirus vectors). Prior to loading, the peptide may be covalently conjugated to an immunological partner that provides T cell help (e.g., a carrier molecule).

The term "HLA" relates to class I human histocompatibility (HLA) surface antigens. A preferred HLA antigen is HLA-B encoded by alleles on locus B of the HLA complex, the most polymorphic of all the HLA specificities. Like other class I HLA determinants, they are involved in the cellular immune reactivity of cytolytic T lymphocytes. HLA-B according to the present invention comprises HLA-B*1501 and HLA-B*1801.

The term "an amino acid sequence as set forth in any one of SEQ ID NO: 1 to 3" refers to an amino acid sequence shown in SEQ ID NO: 1, SEQ ID NO: 2, and SEQ ID NO: 3, respectively, of the Sequence Listing.

The term "peptide" as used herein comprises "oligopeptides", "polypeptides" and "proteins". Peptides as described herein may be of any length. A peptide described herein preferably comprises a sequence of at least 6, in particular at least 8, at least 10, at least 12, at least 15, at least 20, at least 30 or at least 50, consecutive amino acids. Preferably, the peptide comprises no more than 100, in particular no more than 90, no more than 80, no more than 70, no more than 60, no more than 50, no more than 40, no more than 30, no more than 20, no more than 10, or no more than 8, consecutive amino acids. Preferably, the peptide comprises 9 consecutive amino acids. Additional sequences derived from the native protein from which the antigenic or immunogenic portion of the peptide is derived and/or heterologous sequences may be present, and such sequences may (but need not) possess further immunogenic or antigenic properties.

Preferably, the peptides described according to the invention have been isolated. The term "isolated peptide" means that the peptide has been separated from its natural environment. An isolated peptide may be in an essentially purified state. The term "essentially purified" means that the peptide is essentially free of other substances with which it is associated in nature or in vivo.

A peptide "variant" as used herein, comprises a peptide that differs from a peptide from which it is derived in one or more substitutions, deletions, additions and/or insertions of amino acids.

Such variants may be those that exist naturally, or those in which one or more mutation(s) has been introduced.

Peptide variants encompassed by the present invention include those exhibiting at least about 70%, 75%, 80%, 85%, 90%, 91%, 92%, 93%, 94%, 95%, 96%, 97%, 98%, or 99% or more identity to the peptide sequences disclosed herein.

The term "percentage identity" is intended to denote a percentage of nucleotides or of amino acid residues which are identical between the two sequences to be compared, obtained after the best alignment, this percentage being purely statistical and the differences between the two sequences being distributed randomly and over their entire length. Sequence comparisons between two nucleotide or amino acid sequences are conventionally carried out by comparing these sequences after having aligned them optimally, said comparison being carried out be segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and. Wunsch, 1970, J. Mol. Biol. 48, 443, by means of the similarity search method of Pearson and Lipman, 1988, Proc. Natl Acad. Sci. USA 85, 2444, by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity between two nucleic acid or amino acid sequences is determined by comparing these two sequences aligned in an optimal manner in which the nucleic acid or amino acid sequence to be compared may comprise additions or deletions compared to the reference sequence for optimal alignment between these two sequences. The percentage identity is calculated by determining the number of identical positions for which the nucleotide or the amino acid residue is identical between the two sequences, dividing this number of positions compared and multiplying the result obtained by 100 so as to obtain the percentage identity between these two sequences.

For example, program BLAST "BLAST 2 sequences" can be used which is obtained from website http;//www.ncbi.nlm.nih.gov/gorf/b12.html, wherein parameter is default (particularly, open gap penalty is 5, extension gap penalty is 2; matrix is provided by such as program "blosum 62"), the percentage of identity between two sequences to be aligned is directly calculated by the program.

Preferably, a variant contains conservative substitutions. A "conservative substitution" is one in which an amino acid is substituted for another amino acid that has similar properties, such that one skilled in the art of peptide chemistry would expect the secondary structure, charge, and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes.

A peptide "variant" as used herein, also comprises retro-inverse peptides or pseudopeptides analogous to the peptide from which it is derived, which instead of the -CO-NH- peptide bonds include -NH-CO- bonds (Meziere et al., 1997, J. Immunol. 159, 3230-3237). Retro-inverse peptides are much more resistant to proteolysis but can maintain the ability to bind MHC molecules and activate T cells.

According to the invention, a "variant" of a peptide also comprise single or multiple substitutions, deletions and/or additions of any molecules associated with the peptide, such as carbohydrates, lipids and/or proteins. The term "variant" also extends to all functional chemical equivalents of a peptide.

The term "a variant having substantially identical properties" means that the variant has a functional property of the peptide from which it is derived. Thus, a variant may exhibit substantially the same structure, function, antigenicity, immunogenicity etc., as the peptide from which it is derived.

Preferably, if a peptide is able to bind to a HLA molecule such as HLA-B, a variant of said peptide maintains the ability to bind to bind to a HLA molecule and, where appropriate, generate an immune response. This immune response may be based on stimulating cytotoxic T cells. In one embodiment the ability of a variant to bind to a HLA molecule is improved. Positions 2 and 9 of an HLA-binding nonamer are typically anchor residues. Modifications of these and other residues involved in binding may enhance binding without altering CTL recognition (for example, see Tourdot et al., 1997, J. Immunol. 159, 2391-2398).

Those amino acid residues that are not essential to interact with a T cell receptor can be modified by replacement with another amino acid whose incorporation does not substantially effect T cell reactivity and does not eliminate binding to the relevant HLA.

Preferably, the antigenicity and/or immunogenicity of the variant peptide are not substantially diminished. In other words, the ability of a variant to react with antigen-specific antisera may be enhanced or unchanged, or may be diminished by less than 50%, and preferably less than 20%, relative to the peptide from which it is derived. Such variants may generally be identified by modifying a peptide sequence and evaluating the reactivity of the modified peptide with antigen-specific antibodies or antisera as described herein.

A variant will preferably react with antisera and/or T cells at a level that is not substantially less than or greater than the reactivity of the peptide from which the variant is derived (e.g., in an ELISA and/or T cell reactivity assay). Such screens may generally be performed using methods well known to those of ordinary skill in the art, such as those described in Harlow and Lane, Antibodies: A Laboratory Manual, Cold Spring Harbor Laboratory, 1988. For example, a peptide may be immobilized on a solid support and contacted with patient sera to allow binding of antibodies within the sera to the immobilized peptide. Unbound sera may then be removed and bound antibodies detected using, for example, I-labeled Protein A.

In a particular preferred embodiments of the "variants" described herein the variation is present in the portion comprising the amino acid sequence as set forth in any one of SEQ ID NO: 1 to 3. In this embodiment, the present invention relates to peptides comprising an amino acid sequence as set forth in any one of SEQ ID NO: 1 to 3 or a variant of said sequence.

Peptides may be prepared using any of a variety of well known techniques. Peptides described according to the invention may be isolated from biological samples such as tissue or cell homogenates and may also be expressed recombinantly in a multiplicity of pro- or eukaryotic expression systems.

Recombinant peptides encoded by DNA sequences may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant peptide. Suitable host cells include prokaryotes, yeast, and higher eukaryotic cells, such as mammalian cells and plant cells. Preferably, the host cells employed are E. coli, yeast or a mammalian cell line such as COS or CHO. Supernatants from suitable host/vector systems which secrete recombinant peptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant peptide.

Peptides and variants having less than about 100 amino acids, and generally less than about 50 amino acids, may also be generated by synthetic means, using techniques well known to those of ordinary skill in the art. For example, such peptides may be synthesized using any of the commercially available solid-phase techniques, such as the Merrifield solid-phase synthesis method, where amino acids are sequentially added to a growing amino acid chain. See Merrifield, 1963, J. Am. Chem. Soc. 85, 2149-2146. Equipment for automated synthesis of polypeptides is commercially available from suppliers such as Perkin Elmer/Applied BioSystems Division (Foster City, Calif.), and may be operated according to the manufacturer's instructions.

The peptides and variants described herein can be obtained, for instance, by designing peptides conformable to the HLA-B-binding motif based on the amino acid sequences set forth in any one of SEQ ID NO: 1 to 3 of the Sequence Listing, and then selecting from the designed peptides that are recognized by CTL in an HLA-B-restricted manner. The peptides may be those that bind to HLA-B and are presented on the surface of the antigen-presenting cells, and the peptides have the characteristics of an antigen epitope that is recognized by CTL.

Within certain specific embodiments, a peptide may be a fusion protein that comprises multiple sequences as described herein, or that comprises at least one sequence as described herein and an unrelated sequence. A fusion partner may, for example, assist in providing T helper epitopes (an immunological fusion partner), preferably T helper epitopes recognized by humans, or may assist in expressing the protein (an expression enhancer) at higher yields than the native recombinant peptide. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the peptide.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the peptide components may be assembled separately, and ligated into an appropriate expression vector. The 3' end of the DNA sequence encoding one peptide component is ligated, with or without a peptide linker, to the 5' end of a DNA sequence encoding the second peptide component so that the reading frames of the sequences are in phase. This permits translation into a single fusion protein that retains the biological activity of both component peptides.

A peptide linker sequence may be employed to separate the first and second peptide components by a distance sufficient to ensure that each peptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second peptides; and (3) the lack of hydrophobic or charged residues that might react with the peptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., 1985, Gene 40, 39-46; Murphy et al., 1986, Proc. Natl. Acad. Sci. USA 83, 8258-8262, U.S. Pat. No. 4,935,233 and U.S. Pat. No. 4,751,180. The linker sequence may generally be from 1 to about 50 amino acids in length. Linker sequences are not required when the first and second peptides have non-essential N-terminal amino acid regions that can be used to separate the functional domains and prevent steric interference.

Fusion proteins may comprise a peptide as described herein together with an unrelated immunogenic protein. Preferably the immunogenic protein is capable of eliciting a recall response. Examples of such proteins include tetanus, tuberculosis and hepatitis proteins (see, for example, Stoute et al., 1997, New Engl. J. Med., 336, 86-91).

The expression "peptide is not a naturally occurring virus protein" as used herein means that the peptide may not be found in a naturally occurring virus, preferably not found in a virus of the genus Orthopoxvirus. However, this expression does not exclude the possibility that the peptide forms part of a protein present in a naturally occurring virus.

According to the invention, a nucleic acid is preferably deoxyribonucleic acid (DNA) or ribonucleic acid (RNA) or a variant thereof, and may comprise genomic DNA, cDNA, mRNA, recombinantly produced and chemically synthesized molecules. According to the invention, a nucleic acid may be present as a single-stranded or double-stranded and linear or covalently circularly closed molecule.

"Variant" of a nucleic acid means according to the invention that single or multiple nucleotide substitutions, deletions and/or additions are present in said nucleic acid. Furthermore, the term "variant" also comprises chemical derivatization of a nucleic acid on a nucleotide base, on the sugar or on the phosphate. The term "variant" also comprises nucleic acids which contain nucleotides and nucleotide analogs not occurring naturally.

The nucleic acids described herein have preferably been isolated. The term "isolated nucleic acid" means according to the invention that the nucleic acid was (i) amplified in vitro, for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid which is available for manipulation by recombinant DNA techniques.

A nucleic acid is "complementary" to another nucleic acid if the two sequences are capable of hybridizing and forming a stable duplex with one another, with hybridization preferably being carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH₂PO₄ (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 × SSC at room temperature and then in 0.1-0.5 × SSC/0.1 × SDS at temperatures of up to 68°C.

According to the invention, complementary nucleic acids have at least 40%, in particular at least 50%, at least 60%, at least 70%, at least 80%, at least 90% and preferably at least 95%, at least 98 or at least 99%, identical nucleotides.

Nucleic acids coding for peptide may, according to the invention, be present alone or in combination with other nucleic acids, in particular heterologous nucleic acids. In preferred embodiments, a nucleic acid is functionally linked to expression control sequences or regulatory sequences which may be homologous or heterologous with respect to said nucleic acid. A coding sequence and a regulatory sequence are "functionally" linked to one another, if they are covalently linked to one another in such a way that expression or transcription of said coding sequence is under the control or under the influence of said regulatory sequence. If the coding sequence is to be translated into a functional protein, then, with a regulatory sequence functionally linked to said coding sequence, induction of said regulatory sequence results in transcription of said coding sequence, without causing a frame shift in the coding sequence or said coding sequence not being capable of being translated into the desired protein or peptide.

The term "expression control sequence" or "regulatory sequence" comprises according to the invention promoters, enhancers and other control elements which regulate expression of a gene. In particular embodiments of the invention, the expression control sequences can be regulated. The exact structure of regulatory sequences may vary as a function of the species or cell type, but generally comprises 5'untranscribed and 5'untranslated sequences which are involved in initiation of transcription and translation, respectively, such as TATA box, capping sequence, CAAT sequence, and the like. More specifically, 5'untranscribed regulatory sequences comprise a promoter region which includes a promoter sequence for transcriptional control of the functionally linked gene. Regulatory sequences may also comprise enhancer sequences or upstream activator sequences.

According to the invention, a nucleic acid may furthermore be present in combination with another nucleic acid which codes for a peptide controlling secretion of the peptide encoded by said nucleic acid from a host cell. According to the invention, a nucleic acid may also be present in combination with another nucleic acid which codes for a peptide causing the encoded peptide to be anchored on the cell membrane of the host cell or compartmentalized into particular organelles of said cell.

In a preferred embodiment, a recombinant DNA molecule according to the invention is a vector, where appropriate with a promoter, which controls expression of a nucleic acid, for example a nucleic acid coding for a peptide antigen of the invention. The term "vector" is used here in its most general meaning and comprises any intermediary vehicle for a nucleic acid which enables said nucleic acid, for example, to be introduced into prokaryotic and/or eukaryotic cells and, where appropriate, to be integrated into a genome. Vectors of this kind are preferably replicated and/or expressed in the cells. An intermediary vehicle may be adapted, for example, to the use in electroporation, in bombardment with microprojectiles, in liposomal administration, in the transfer with the aid of agrobacteria or in insertion via DNA or RNA viruses. Vectors comprise plasmids, phagemids or viral genomes.

The nucleic acids coding for a peptide as described herein may be used for transfection of host cells. Nucleic acids here mean both recombinant DNA and RNA. Recombinant RNA may be prepared by in-vitro transcription of a DNA template. Furthermore, it may be modified by stabilizing sequences, capping and polyadenylation prior to application. According to the invention, the term "host cell" relates to any cell which can be transformed or transfected with an exogenous nucleic acid. The term "host cells" comprises according to the invention prokaryotic (e.g. E. coli) or eukaryotic cells (e.g. dendritic cells, monocytes, B cells, CHO cells, COS cells, K562 cells, yeast cells and insect cells). Particular preference is given to mammalian cells such as cells from humans, mice, hamsters, pigs, goats, primates. The cells may be derived from a multiplicity of tissue types and comprise primary cells and cell lines. Specific examples comprise keratinocytes, peripheral blood leukocytes, stem cells of the bone marrow and embryonic stem cells. In further embodiments, the host cell is an antigen-presenting cell, in particular a dendritic cell, monocyte or a macrophage. A nucleic acid may be present in the host cell in the form of a single copy or of two or more copies and, in one embodiment, is expressed in the host cell.

According to the invention, the term "expression" is used in its most general meaning and comprises the production of RNA or of RNA and protein. It also comprises partial expression of nucleic acids. Furthermore, expression may be carried out transiently or stably. Preferred expression systems in mammalian cells comprise pcDNA3.1 and pRc/CMV (Invitrogen, Carlsbad, CA), which contain a selective marker such as a gene imparting resistance to G418 (and thus enabling stably transfected cell lines to be selected) and the enhancer-promoter sequences of cytomegalovirus (CMV).

In those cases of the invention in which an HLA molecule presents a peptide, an expression vector may also comprise a nucleic acid sequence coding for said HLA molecule. The nucleic acid sequence coding for the HLA molecule may be present on the same expression vector as the nucleic acid coding for the peptide antigen, or both nucleic acids may be present on different expression vectors. In the latter case, the two expression vectors may be cotransfected into a cell. If a host cell expresses neither the peptide antigen nor the HLA molecule, both nucleic acids coding therefor are transfected into the cell either on the same expression vector or on different expression vectors. If the cell already expresses the HLA molecule, only the nucleic acid sequence coding for the peptide antigen can be transfected into the cell.

In another aspect, the present invention provides methods for inducing protective immunity against Orthopoxvirus infection in a patient.

As used herein, a "patient" refers to any warm-blooded animal, such as a human, a nonhuman primate or another animal, in particular a mammal such as a cow, horse, pig, sheep, goat, dog, cat or a rodent such as a mouse and rat, preferably a human. A patient may be afflicted with a disease, or may be free of detectable disease and/or infection. In other words, protective immunity may be induced to prevent or treat Orthopoxvirus infection.

Pharmaceutical compositions may comprise one or more peptides and a physiologically acceptable carrier. Vaccines may comprise one or more peptides and an immunostimulant, such as an adjuvant or a liposome (into which the peptide is incorporated). Such pharmaceutical compositions and vaccines may also contain other antigens, either incorporated into a combination peptide or present within a separate peptide.

Alternatively, a pharmaceutical composition may contain nucleic acids encoding one or more peptides as described above, such that the peptide is generated in situ. In such pharmaceutical compositions, the nucleic acids may be present within any of a variety of delivery systems known to those of ordinary skill in the art, including nucleic acid expression systems, bacterial and viral expression systems. Appropriate nucleic acid expression systems contain the necessary nucleic acid sequences for expression in the patient (such as a suitable promoter and terminating signal). Bacterial delivery systems involve the administration of a bacterium (such as Bacillus-Calmette-Guerrin) that expresses an immunogenic portion of the peptide on its cell surface. In a preferred embodiment, the nucleic acids may be introduced using a viral expression system (e.g., vaccinia or other pox virus, retrovirus, or adenovirus), which may involve the use of a non-pathogenic (defective) virus. Techniques for incorporating nucleic acids into such expression systems are well known to those of ordinary skill in the art. The nucleic acids may also be administered as "naked" plasmid vectors as described, for example, in Ulmer et al., 1993, Science 259, 1745-1749, and reviewed by Cohen, 1993, Science 259, 1691-1692. Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle in vitro and in vivo is a liposome (i.e., an artificial membrane vesicle). The uptake of naked nucleic acids may be increased by incorporating the nucleic acids into and/or onto biodegradable beads, which are efficiently transported into the cells. The preparation and use of such systems is well known in the art.

In a related aspect, a nucleic acid composition as described above may be administered simultaneously with or sequentially to either a peptide as described in the present invention or a known antigen. For example, administration of nucleic acids encoding a peptide as described herein, either "naked" or in a delivery system as described above, may be followed by administration of an antigen in order to enhance the protective immune effect.

Peptides and nucleic acids disclosed herein may also be employed in adoptive immunotherapy for the treatment of Orthopoxvirus infection. Adoptive immunotherapy may be broadly classified into either active or passive immunotherapy. In active immunotherapy, treatment relies on the in vivo stimulation of the endogenous host immune system with the administration of immune response-modifying agents (for example, vaccines, bacterial adjuvants, and/or cytokines). In passive immunotherapy, treatment involves the delivery of biologic reagents with established immune reactivity (such as effector cells or antibodies) that can directly or indirectly mediate anti-viral effects and does not necessarily depend on an intact host immune system. Examples of effector cells include T lymphocytes (for example, CD8+ cytotoxic T lymphocyte, CD4+ T helper), killer cells (such as Natural Killer cells, lymphokine-activated killer cells), B cells, or antigen presenting cells (such as dendritic cells and macrophages) expressing the antigens. The peptides disclosed herein may also be used to generate antibodies or anti-idiotypic antibodies (as in U.S. Pat. No. 4,918,164), for passive immunotherapy.

The predominant method of procuring adequate numbers of T cells for adoptive immunotherapy is to grow immune T cells in vitro. Culture conditions for expanding single antigen-specific T cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive peptides described herein may be used to rapidly expand antigen-specific T cell cultures in order to generate sufficient number of cells for immunotherapy. In particular, antigen-presenting cells, such as dendritic cells, macrophages, monocytes, fibroblasts, or B-cells, may be pulsed with immunoreactive peptides, or nucleic acid sequence(s) may be introduced into antigen presenting cells, using a variety of standard techniques well known in the art. For example, antigen presenting cells may be transfected or transduced with a nucleic acid sequence, wherein said sequence contains a promoter region appropriate for increasing expression, and can be expressed as part of a recombinant virus or other expression system. Several viral vectors may be used to transduce an antigen presenting cell, including pox virus, vaccinia virus, and adenovirus; also, antigen presenting cells may be transfected with nucleic acid sequences disclosed herein by a variety of means, including gene-gun technology, lipid-mediated delivery, electroporation, osmotic shock, and particle delivery mechanisms, resulting in efficient and acceptable expression levels as determined by one of ordinary skill in the art. For cultured T cells to be effective in therapy, the cultured T cells must be able to grow and distribute widely and to survive long term in vivo. Studies have demonstrated that cultured T cells can be induced to grow in vivo and to survive long term in substantial numbers by repeated stimulation with antigen supplemented with IL-2 (see, for example, Cheever, M. et al., 1997, Immunological Reviews 157, 177).

The peptides disclosed herein may also be employed to generate and/or isolate virus-reactive T cells, which can then be administered to the patient. In one technique, antigen-specific T cell lines may be generated by in vivo immunization with short peptides corresponding to immunogenic portions of the disclosed peptides. The resulting antigen specific CD8+ or CD4+ T cell clones may be isolated from the patient, expanded using standard tissue culture techniques, and returned to the patient.

Alternatively, peptides corresponding to immunogenic portions of the peptides described herein may be employed to generate virus-reactive T cell subsets by selective in vitro stimulation and expansion of autologous T cells to provide antigen-specific T cells which may be subsequently transferred to the patient as described, for example, by Chang et al. (1996, Crit. Rev. Oncol. Hematol. 22(3), 213). Cells of the immune system, such as T cells, may be isolated from the peripheral blood of a patient, using a commercially available cell separation system, such as Isolex(TM) System, available from Nexell Therapeutics, Inc. Irvine, Calif. The separated cells are stimulated with one or more of the immunoreactive peptides contained within a delivery vehicle, such as a microsphere, to provide antigen-specific T cells. The population of antigen-specific T cells is then expanded using standard techniques and the cells are administered back to the patient.

In another method of selecting antigen-specific cytotoxic T lymphocytes, fluorogenic tetramers of MHC class I molecule/peptide complexes are used for detecting specific clones of cytotoxic T lymphocytes (Altman et al., Science 274:94-96, 1996; Dunbar et al., Curr. Biol. 8:413-416, 1998). Soluble MHC class I molecules are folded in vitro in the presence of β₂ microglobulin and a peptide antigen binding to said class I molecule. The MHC/peptide complexes are purified and then labeled with biotin. Tetramers are formed by mixing the biotinylated peptide-MHC complexes with labeled avidin (e.g. phycoerythrin) in a molar ratio of 4:1. Tetramers are then contacted with cytotoxic T lymphocytes such as peripheral blood or lymph nodes. The tetramers bind to cytotoxic T lymphocytes which recognize the peptide antigen/MHC class I complex. Cells which are bound to the tetramers may be sorted by fluorescence-controlled cell sorting to isolate reactive cytotoxic T lymphocytes. The isolated cytotoxic T lymphocytes may then be propagated in vitro.

In other embodiments, T cell and/or antibody receptors specific for the peptides disclosed herein can be cloned, expanded, and transferred into other vectors or effector cells for use in adoptive immunotherapy. In particular, T cells may be transfected with the appropriate genes to express the variable domains from virus specific monoclonal antibodies as the extracellular recognition elements and joined to the T cell receptor signaling chains, resulting in T cell activation, specific lysis, and cytokine release. This enables the T cell to redirect its specificity in an MHC-independent manner. See for example, Eshhar, Z., 1997, Cancer Immunol Immunother, 45(3-4), 131-6, and Hwu, P. et al., 1995, Cancer Res, 55(15), 3369-73. Another embodiment may include the transfection of vaccinia virus antigen specific alpha and beta T cell receptor chains into alternate T cells, as in Cole, D. J. et al., 1995, Cancer Res, 55(4), 748-52.

In a further embodiment, syngeneic or autologous dendritic cells may be pulsed with peptides corresponding to at least an immunogenic portion of a peptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate disease in a murine model has been demonstrated by Cheever et al., 1997, Immunological Reviews 157, 177). Additionally, vectors expressing the disclosed nucleic acids may be introduced into stem cells taken from the patient and clonally propagated in vitro for autologous transplant back into the same patient.

The aforementioned peptides may be used singly or used in combination of two or more in order to induce and/or activate CTL. Since CTL are a plurality of cell groups that recognize various antigens, it is recommended that preferably two or more of these be used in combination.

Within certain aspects, peptides, nucleic acids, T cells and/or binding agents disclosed herein may be incorporated into pharmaceutical compositions or immunogenic compositions (i.e., vaccines). Alternatively, a pharmaceutical composition may comprise an antigen-presenting cell (e.g. a dendritic cell) transfected with a nucleic acid such that the antigen presenting cell expresses a peptide. Pharmaceutical compositions comprise one or more such compounds and a physiologically acceptable carrier. Vaccines may comprise one or more such compounds and an immunostimulant. Pharmaceutical compositions and vaccines within the scope of the present invention may also contain other compounds, which may be biologically active or inactive.

A pharmaceutical composition or vaccine may contain nucleic acids encoding one or more of the peptides as described above, such that the peptide is generated in situ. As noted above, the nucleic acids may be present within any of a variety of delivery systems known to those of ordinary skill in the art, including nucleic acid expression systems, bacteria and viral expression systems. Numerous gene delivery techniques are well known in the art, such as those described by Rolland, 1998, Crit. Rev. Therap. Drug Carrier Systems 15, 143-198, and references cited therein. Appropriate nucleic acid expression systems contain the necessary DNA sequences for expression in the patient (such as a suitable promoter and terminating signal). Bacterial delivery systems involve the administration of a bacterium (such as Bacillus-Calmette-Guerrin) that expresses an immunogenic portion of the peptide on its cell surface or secretes such an epitope.

In a preferred embodiment, the DNA may be introduced using a viral expression system (e.g., vaccinia or other pox virus, retrovirus, adenovirus, baculovirus, togavirus, bacteriophage, and the like), which often involves the use of a non-pathogenic (defective), replication competent virus.

For example, many viral expression vectors are derived from viruses of the retroviridae family. This family includes the murine leukemia viruses, the mouse mammary tumor viruses, the human foamy viruses, Rous sarcoma virus, and the immunodeficiency viruses, including human, simian, and feline. Considerations when designing retroviral expression vectors are discussed in Comstock et al. (1997).

A large number of adenovirus-based expression vectors have been developed, primarily due to the advantages offered by these vectors in gene therapy applications. Adenovirus expression vectors and methods of using such vectors are the subject of a number of United States patents, including U.S. Pat. No. 5,698,202, U.S. Pat. No. 5,616,326, U.S. Pat. No. 5,585,362, and U.S. Pat. No. 5,518,913, all incorporated herein by reference.

Poxviruses are widely used for the expression of heterologous genes in mammalian cells. Over the years, the vectors have been improved to allow high expression of the heterologous gene and simplify the integration of multiple heterologous genes into a single molecule. In an effort to diminish cytopathic effects and to increase safety, vaccinia virus mutant and other poxviruses that undergo abortive infection in mammalian cells are receiving special attention (Oertli et al., 1997, Clin. Exp. Immunol. 110, 144-149). The use of poxviruses as expression vectors is reviewed in Carroll and Moss (Curr Opin Biotechnol. 8, 573-577, 1997).

Various methods may be used in order to introduce according to the invention nucleic acids into cells in vitro or in vivo. Methods of this kind comprise transfection of nucleic acid CaPO₄ precipitates, transfection of nucleic acids associated with DEAE, transfection or infection with the above viruses carrying the nucleic acids of interest, liposome-mediated transfection, and the like. In particular embodiments, preference is given to directing the nucleic acid to particular cells. In such embodiments, a carrier used for administering a nucleic acid to a cell (e.g. a retrovirus or a liposome) may have a bound target control molecule. For example, a molecule such as an antibody specific for a surface membrane protein on the target cell or a ligand for a receptor on the target cell may be incorporated into or attached to the nucleic acid carrier. Preferred antibodies comprise antibodies which bind selectively an antigen. If administration of a nucleic acid via liposomes is desired, proteins binding to a surface membrane protein associated with endocytosis may be incorporated into the liposome formulation in order to make target control and/or uptake possible. Such proteins comprise capsid proteins or fragments thereof which are specific for a particular cell type, antibodies to proteins which are internalized, proteins addressing an intracellular site, and the like.

The pharmaceutical compositions described according to the invention may also be used as vaccines for immunization. According to the invention, the terms "immunization" or "vaccination" mean an increase in or activation of an immune response to an antigen. It is possible to use animal models for testing an immunizing effect.

Vaccines may comprise or may be administered together with one or more adjuvants for inducing an immune response or for increasing an immune response. An adjuvant is a substance which is incorporated into the antigen or administered together with the latter and which enhances the immune response. Adjuvants may enhance the immune response by providing an antigen reservoir (extracellularly or in macrophages), activating macrophages and stimulating particular lymphocytes. Adjuvants are known and comprise in a nonlimiting way monophosphoryl lipid A (MPL, SmithKline Beecham), saponin such as QS21 (SmithKline Beecham), DQS21 (SmithKline Beecham; WO 96/33739), QS7, QS17, QS18 and QS-L1 (So et al., 1997, Mol. Cells 7, 178-186,), incomplete Freund's adjuvant, complete Freund's adjuvant, vitamin E, montanide, alum, CpG oligonucleotides (cf. Krieg et al., 1995, Nature 374, 546-9) and various water-in-oil emulsions prepared from biologically degradable oils such as squalene and/or tocopherol. Preferably, the peptides are administered in a mixture with DQS21/MPL. The ratio of DQS21 to MPL is typically about 1:10 to 10:1, preferably about 1:5 to 5:1 and in particular about 1:1. For administration to humans, a vaccine formulation typically contains DQS21 and MPL in a range from about 1 µg to about 100 µg.

Other substances which stimulate an immune response of the patient may also be administered. It is possible, for example, to use cytokines in a vaccination, owing to their regulatory properties on lymphocytes. Such cytokines comprise, for example, interleukin-12 (IL-12) which was shown to increase the protective actions of vaccines (cf. Science 268, 1432-1434, 1995), GM-CSF and IL-18.

There are a number of compounds which enhance an immune response and which therefore may be used in a vaccination. Said compounds comprise costimulating molecules provided in the form of proteins or nucleic acids. Examples of such costimulating molecules are B7-1 and B7-2 (CD80 and CD86, respectively) which are expressed on dendritic cells (DC) and interact with the CD28 molecule expressed on the T cells. This interaction provides a costimulation (signal 2) for an antigen/MHC/TCR-stimulated (signal 1) T cell, thereby enhancing propagation of said T cell and the effector function. B7 also interacts with CTLA4 (CD 152) on T cells, and studies involving CTLA4 and B7 ligands demonstrate that B7-CTLA4 interaction can enhance antitumor immunity and CTL propagation (Zheng, P. et al., 1998, Proc. Natl. Acad. Sci. USA 95(11), 6284-6289).

A complete activation of cytotoxic T lymphocytes and a complete effector function require an involvement of T helper cells via interaction between the CD40 ligand on said T helper cells and the CD40 molecule expressed by dendritic cells (Ridge et al., 1998, Nature 393, 474, Bennett et al., 1998, Nature 393, 478, Schönberger et al., 1998, Nature 393, 480). The mechanism of this costimulating signal probably relates to the increase in B7 production and associated IL-6/IL-12 production by said dendritic cells (antigen-presenting cells). CD40-CD40L interaction thus complements the interaction of signal 1 (antigen/MHC-TCR) and signal 2 (B7-CD28).

The use of anti-CD40 antibodies for stimulating dendritic cells would be expected to directly enhance a response to antigens which are usually outside the range of an inflammatory response or which are presented by nonprofessional antigen-presenting cells. In these situations, T helper and B7-costimulating signals are not provided. This mechanism could be used in connection with therapies based on antigen-pulsed dendritic cells or in situations in which T helper epitopes have not been defined in known TRA precursors.

The therapeutic compositions of the invention may be administered in pharmaceutically compatible preparations. Such preparations may usually contain pharmaceutically compatible concentrations of salts, buffer substances, preservatives, carriers, supplementing immunity-enhancing substances such as adjuvants, CpG and cytokines and, where appropriate, other therapeutically active compounds.

The therapeutically active compounds of the invention may be administered via any conventional route, including by injection or infusion. The administration may be carried out, for example, orally, intravenously, intraperitonealy, intramuscularly, subcutaneously or transdermally. Preferably, antibodies are therapeutically administered by way of a lung aerosol.

The compositions of the invention are administered in effective amounts. An "effective amount" refers to the amount which achieves a desired reaction or a desired effect alone or together with further doses. In the case of treatment of a particular disease or of a particular condition, the desired reaction relates to inhibition of the course of the disease. This comprises slowing down the progress of the disease and, in particular, interrupting the progress of the disease. The desired reaction in a treatment of a disease or of a condition may also be delay of the onset or a prevention of the onset of said disease or said condition.

An effective amount of a composition of the invention will depend on the condition to be treated, the severity of the disease, the individual parameters of the patient, including age, physiological condition, size and weight, the duration of treatment, the type of an accompanying therapy (if present), the specific route of administration and similar factors.

The pharmaceutical compositions of the invention are preferably sterile and contain an effective amount of the therapeutically active substance to generate the desired reaction or the desired effect.

The doses administered of the compositions of the invention may depend on various parameters such as the type of administration, the condition of the patient, the desired period of administration, etc. In the case that a reaction in a patient is insufficient with an initial dose, higher doses (or effectively higher doses achieved by a different, more localized route of administration) may be used.

Generally, doses of the peptide of from 1 ng to 1 mg, preferably from 10 ng to 100 µg, are formulated and administered for a treatment or for generating or increasing an immune response. If the administration of nucleic acids (DNA and RNA) is desired, doses of from 1 ng to 0.1 mg are formulated and administered.

The pharmaceutical compositions of the invention are generally administered in pharmaceutically compatible amounts and in pharmaceutically compatible compositions. The term "pharmaceutically compatible" refers to a nontoxic material which does not interact with the action of the active component of the pharmaceutical composition. Preparations of this kind may usually contain salts, buffer substances, preservatives, carriers and, where appropriate, other therapeutically active compounds. When used in medicine, the salts should be pharmaceutically compatible. However, salts which are not pharmaceutically compatible may used for preparing pharmaceutically compatible salts and are included in the invention.

Pharmacologically and pharmaceutically compatible salts of this kind comprise in a nonlimiting way those prepared from the following acids: hydrochloric, hydrobromic, sulfuric, nitric, phosphoric, maleic, acetic, salicylic, citric, formic, malonic, succinic acids, and the like. Pharmaceutically compatible salts may also be prepared as alkali metal salts or alkaline earth metal salts, such as sodium salts, potassium salts or calcium salts.

A pharmaceutical composition of the invention may comprise a pharmaceutically compatible carrier. According to the invention, the term "pharmaceutically compatible carrier" refers to one or more compatible solid or liquid fillers, diluents or encapsulating substances, which are suitable for administration to humans. The term "carrier" refers to an organic or inorganic component, of a natural or synthetic nature, in which the active component is combined in order to facilitate application. The components of the pharmaceutical composition of the invention are usually such that no interaction occurs which substantially impairs the desired pharmaceutical efficacy.

The pharmaceutical compositions of the invention may contain suitable buffer substances such as acetic acid in a salt, citric acid in a salt, boric acid in a salt and phosphoric acid in a salt.

The pharmaceutical compositions may, where appropriate, also contain suitable preservatives such as benzalkonium chloride, chlorobutanol, paraben and thimerosal.

The pharmaceutical compositions are usually provided in a uniform dosage form and may be prepared in a manner known per se. Pharmaceutical compositions of the invention may be in the form of capsules, tablets, lozenges, suspensions, syrups, elixir or in the form of an emulsion, for example.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

In another aspect, the present invention provides methods for diagnosing Orthopoxvirus infection. In this aspect, methods are provided for detecting Orthopoxvirus infection in a biological sample, using one or more of the peptides described herein, either alone or in combination.

As used herein, a "biological sample" is any sample obtained from a patient. Preferably, the sample is whole blood, sputum, serum, plasma, saliva, cerebrospinal fluid or urine. The biological sample to be detected may be derived from human beings and other primates, such as baboon, ape, monkey, etc.; economic animals, such as, bovine, caprine, swine, rabbit, murine, as well as pets, such as feline, canine, etc. More preferably, the sample is a blood, serum or plasma sample obtained from a patient.

The peptides or variants described herein are preferably used in an assay to determine the presence or absence of antibodies or T cells to the peptides or variants in the sample. The presence of such antibodies or T cells indicates previous sensitization to virus antigens which may be indicative of virus infection.

A variety of assay formats are known to those of ordinary skill in the art for using one or more peptides to detect antibodies in a sample. See, e.g., Harlow and Lane, Antibodies: A Laboratory Manual, Cold Spring Harbor Laboratory, 1988, which is incorporated herein by reference. In a preferred embodiment, the assay involves the use of peptide immobilized on a solid support to bind to and remove the antibody from the sample. The bound antibody may then be detected using a detection reagent that contains a reporter group. Suitable detection reagents include antibodies that bind to the antibody/peptide complex and free peptide labeled with a reporter group (e.g., in a semi-competitive assay). Alternatively, a competitive assay may be utilized, in which an antibody that binds to the peptide is labeled with a reporter group and allowed to bind to the immobilized antigen after incubation of the antigen with the sample. The extent to which components of the sample inhibit the binding of the labeled antibody to the peptide is indicative of the reactivity of the sample with the immobilized peptide.

The solid support may be any solid material known to those of ordinary skill in the art to which the antigen may be attached. For example, the solid support may be a test well in a microtiter plate, or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Pat. No. 5,359,681.

The peptides may be bound to the solid support using a variety of techniques known to those of ordinary skill in the art. In this context, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. In such cases, adsorption may be achieved by contacting the peptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of peptide ranging from about 10 ng to about 1 µg, and preferably about 100 ng, is sufficient to bind an adequate amount of antigen.

Covalent attachment of peptide to a solid support may generally be achieved by first reacting the support with a bifunctional reagent that will react with both the support and a functional group, such as a hydroxyl or amino group, on the peptide. For example, the peptide may be bound to supports having an appropriate polymer coating using benzoquinone or by condensation of an aldehyde group on the support with an amine and an active hydrogen on the peptide (see, e.g., Pierce Immunotechnology Catalog and Handbook, 1991, at A12-A13).

In certain embodiments, the assay is an enzyme linked immunosorbent assay (ELISA). This assay may be performed by first contacting a peptide antigen that has been immobilized on a solid support, commonly the well of a microtiter plate, with the sample, such that antibodies to the peptide within the sample are allowed to bind to the immobilized peptide. Unbound sample is then removed from the immobilized peptide and a detection reagent capable of binding to the immobilized antibody-peptide complex is added. The amount of detection reagent that remains bound to the solid support is then determined using a method appropriate for the specific detection reagent.

More specifically, once the peptide is immobilized on the support as described above, the remaining protein binding sites on the support are typically blocked. Any suitable blocking agent known to those of ordinary skill in the art, such as bovine serum albumin (BSA) or Tween 20 (Sigma Chemical Co., St. Louis, Mo.) may be employed. The immobilized peptide is then incubated with the sample, and antibody is allowed to bind to the antigen. The sample may be diluted with a suitable dilutent, such as phosphate-buffered saline (PBS) prior to incubation. In general, an appropriate contact time (i.e., incubation time) is that period of time that is sufficient to detect the presence of antibody within an infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20. Detection reagent may then be added to the solid support. An appropriate detection reagent is any compound that binds to the immobilized antibody-peptide complex and that can be detected by any of a variety of means known to those in the art. Preferably, the detection reagent contains a binding agent (such as, for example, Protein A, Protein G, immunoglobulin, lectin or free antigen) conjugated to a reporter group. Preferred reporter groups include enzymes (such as horseradish peroxidase), substrates, cofactors, inhibitors, dyes, radionuclides, luminescent groups, fluorescent groups and biotin. The conjugation of binding agent to reporter group may be achieved using standard methods known to those of ordinary skill in the art. Common binding agents may also be purchased conjugated to a variety of reporter groups from many commercial sources (e.g., Zymed Laboratories, San Francisco, Calif., and Pierce, Rockford, Ill.).

The detection reagent is then incubated with the immobilized antibody-peptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti-virus antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for virus infection. In an alternate preferred embodiment, the cut-off value is determined using a Receiver Operator Curve, according to the method of Sackett et al., Clinical Epidemiology: A Basic Science for Clinical Medicine, Little Brown and Co., 1985, pp. 106-107. Briefly, in this embodiment, the cut-off value may be determined from a plot of pairs of true positive rates (i.e., sensitivity) and false positive rates (100%-specificity) that correspond to each possible cut-off value for the diagnostic test result. The cut-off value on the plot that is the closest to the upper left-hand corner (i.e., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for virus infection.

In a related embodiment, the assay is performed in a rapid flow-through or strip test format, wherein the antigen is immobilized on a membrane, such as nitrocellulose. In the flow-through test, antibodies within the sample bind to the immobilized peptide as the sample passes through the membrane. A detection reagent (e.g., protein A-colloidal gold) then binds to the antibody-peptide complex as the solution containing the detection reagent flows through the membrane. The detection of bound detection reagent may then be performed as described above. In the strip test format, one end of the membrane to which peptide is bound is immersed in a solution containing the sample. The sample migrates along the membrane through a region containing detection reagent and to the area of immobilized peptide. Concentration of detection reagent at the peptide indicates the presence of anti-virus antibodies in the sample. Typically, the concentration of detection reagent at that site generates a pattern, such as a line, that can be read visually. The absence of such a pattern indicates a negative result. In general, the amount of peptide immobilized on the membrane is selected to generate a visually discernible pattern when the biological sample contains a level of antibodies that would be sufficient to generate a positive signal in an ELISA, as discussed above. Preferably, the amount of peptide immobilized on the membrane ranges from about 25 ng to about 11 g, and more preferably from about 50 ng to about 500 ng. Such tests can typically be performed with a very small amount (e.g., one drop) of patient serum or blood.

In another embodiments of the methods for diagnosing Orthopoxvirus infection cells presenting a complex between a MHC molecule and a peptide or variant thereof according to the present invention may be combined with a sample from the patient, which contains cytotoxic T lymphocytes. If the cytotoxic T lymphocytes lyse the cells presenting the complex, it can be assumed that an antigen is presented in the patient and thus, the patient is infected with Orthopoxvirus.

The present invention also enables agents binding to peptides described herein to be isolated, including antibodies or antibody fragments and T cell receptors.

Such binding agents may be used, for example, in screening assays for detecting peptides as described herein and complexes of peptides with their binding partners and in a purification of said peptides and of complexes thereof with their binding partners. Such agents may also be used for inhibiting the activity of virus proteins, for example by binding to such proteins:
"Antibodies" as used herein comprises polyclonal and monoclonal antibodies and fragments thereof and further comprises chimeric or humanized antibodies.

It is known that only a small part of an antibody molecule, the paratope, is involved in binding of the antibody to its epitope (cf. Clark, W.R. (1986), The Experimental Foundations of Modern Immunology, Wiley & Sons, Inc., New York; Roitt, I. (1991), Essential Immunology, 7th Edition, Blackwell Scientific Publications, Oxford). The pFc' and Fc regions are, for example, effectors of the complement cascade but are not involved in antigen binding. An antibody from which the pFc' region has been enzymatically removed or which has been produced without the pFc' region, referred to as F(ab')₂ fragment, carries both antigen binding sites of a complete antibody. Similarly, an antibody from which the Fc region has been enzymatically removed or which has been produced without said Fc region, referred to Fab fragment, carries one antigen binding site of an intact antibody molecule. Furthermore, Fab fragments consist of a covalently bound light chain of an antibody and part of the heavy chain of said antibody, referred to as Fd. The Fd fragments are the main determinants of antibody specificity (a single Fd fragment can be associated with up to ten different light chains, without altering the specificity of the antibody) and Fd fragments, when isolated, retain the ability to bind to an epitope.

Located within the antigen-binding part of an antibody are complementary-determining regions (CDRs) which interact directly with the antigen epitope and framework regions (FRs) which maintain the tertiary structure of the paratope. Both the Fd fragment of the heavy chain and the light chain of IgG immunoglobulines contain four framework regions (FR1 to FR4) which are separated in each case by three complementary-determining regions (CDR1 to CDR3). The CDRs and, in particular, the CDR3 regions and, still more particularly, the CDR3 region of the heavy chain are responsible to a large extent for antibody specificity.

Non-CDR regions of a mammalian antibody are known to be able to be replaced by similar regions of antibodies with the same or a different specificity, with the specificity for the epitope of the original antibody being retained. This made possible the development of "humanized" antibodies in which nonhuman CDRs are covalently linked to human FR and/or Fc/pFc' regions to produce a functional antibody.

WO 92/04381, for example, describes production and use of humanized murine RSV antibodies in which at least part of the murine FR regions have been replaced with FR regions of a human origin. Antibodies of this kind, including fragments of intact antibodies with antigen-binding capability, are often referred to as "chimeric" antibodies.

The invention also provides F(ab')₂, Fab, Fv, and Fd fragments of antibodies, chimeric antibodies, in which the Fc and/or FR and/or CDR1 and/or CDR2 and/or light chain-CDR3 regions have been replaced with homologous human or nonhuman sequences, chimeric F(ab')₂-fragment antibodies in which the FR and/or CDR1 and/or CDR2 and/or light chain-CDR3 regions have been replaced with homologous human or nonhuman sequences, chimeric Fab-fragment antibodies in which the FR and/or CDR1 and/or CDR2 and/or light chain-CDR3 regions have been replaced with homologous human or nonhuman sequences, and chimeric Fd-fragment antibodies in which the FR and/or CDR1 and/or CDR2 regions have been replaced with homologous human or nonhuman sequences. The invention also comprises "single-chain" antibodies.

The invention also comprises polypeptides which bind to the peptides and variants described herein. Polypeptide binding substances of this kind may be provided, for example, by degenerate peptide libraries which may be prepared simply in solution in an immobilized form or as phage-display libraries. It is likewise possible to prepare combinatorial libraries of peptides with one or more amino acids. Libraries of peptids and nonpeptidic synthetic residues may also be prepared.

Phage display may be particularly effective in identifying binding peptides of the invention. In this connection, for example, a phage library is prepared (using, for example, the M13, fd or lambda phages) which presents inserts of from 4 to about 80 amino acid residues in length. Phages are then selected which carry inserts which bind to the peptide. This process may be repeated via two or more cycles of a reselection of phages binding to the peptide. Repeated rounds result in a concentration of phages carrying particular sequences. An analysis of DNA sequences may be carried out in order to identify the sequences of the expressed polypeptides. The smallest linear portion of the sequence binding to the peptide may be determined. The "two-hybrid system" of yeast may also be used for identifying polypeptides which bind to a peptide. Peptides described according to the invention or fragments thereof may be used for screening peptide libraries, including phage-display libraries, in order to identify and select peptide binding partners of the peptide antigens. Such molecules may be used, for example, for screening assays, purification protocols, for interference with the function of the peptide and for other purposes known to the skilled worker.

Antibodies may also be coupled to specific diagnostic substances for displaying cells and tissues expressing particular peptides. They may also be coupled to therapeutically useful substances. Diagnostic substances comprise, in a nonlimiting manner, barium sulfate, iocetamic acid, iopanoic acid, calcium ipodate, sodium diatrizoate, meglumine diatrizoate, metrizamide, sodium tyropanoate and radio diagnostic, including positron emitters such as fluorine-18 and carbon-11, gamma emitters such as iodine-123, technetium-99m, iodine-131 and indium-111, nuclides for nuclear magnetic resonance, such as fluorine and gadolinium.

According to the invention, the term "therapeutically useful substance" means any therapeutic molecule which, as desired, is selectively guided to a cell which expresses one or more target molecules, such as peptides described herein, including radioactive iodine-labeled compounds, toxins, cytostatic or cytolytic drugs, etc. anticancer agents comprising, for example, aminoglutethimide, azathioprine, bleomycin sulfate, busulfan, carmustine, chlorambucil, cisplatin, cyclophosphamide, cyclosporine, cytarabidine, dacarbazine, dactinomycin, daunorubin, doxorubicin, taxol, etoposide, fluorouracil, interferon-α, lomustine, mercaptopurine, methotrexate, mitotane, procarbazine HC1, thioguanine, vinblastine sulfate and vincristine sulfate. Other anticancer agents are described, for example, in Goodman and Gilman, "The Pharmacological Basis of Therapeutics", 8th Edition, 1990, McGraw-Hill, Inc., in particular Chapter 52 (Antineoplastic Agents (Paul Calabresi and Bruce A. Chabner)). Toxins may be proteins such as pokeweed antiviral protein, cholera toxin, pertussis toxin, ricin, gelonin, abrin, diphtheria exotoxin or Pseudomonas exotoxin. Toxin residues may also be high energy-emitting radionuclides such as cobalt-60.

Antibodies may be prepared by any of a variety of techniques known to those of ordinary skill in the art. See, e.g., Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the peptide is initially injected into any of a wide variety of mammals (e.g., mice, rats, rabbits, sheep or goats). In this step, the peptides of this invention may serve as the immunogen without modification. Alternatively, particularly for relatively short peptides, a superior immune response may be elicited if the peptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the peptide may then be purified from such antisera by, for example, affinity chromatography using the polypeptide coupled to a suitable solid support.

Monoclonal antibodies specific for an antigenic peptide of interest may be prepared, for example, using the technique of Kohler and Milstein, Eur. J. Immunol. 6:511-519, 1976, and improvements thereto.

A variety of routes of administration for the antibodies and immunoconjugates may be used. Typically, administration will be intravenous, intramuscular, subcutaneous or in site-specific regions by appropriate methods. It will be evident that the precise dose of the antibody/immunoconjugate will vary depending upon the antibody used, the antigen density, and the rate of clearance of the antibody.

Antibodies may be used in diagnostic tests to detect the presence of Orthopoxvirus antigens using assays similar to those detailed above and other techniques well known to those of skill in the art, thereby providing a method for detecting Orthopoxvirus infection in a patient.

The present invention is further illustrated in details with reference to the following drawings and examples, which should not in any way be interpreted as being limiting to the scope of the present invention.

### BRIEF DESCRIPTION OF THE DRAWINGS

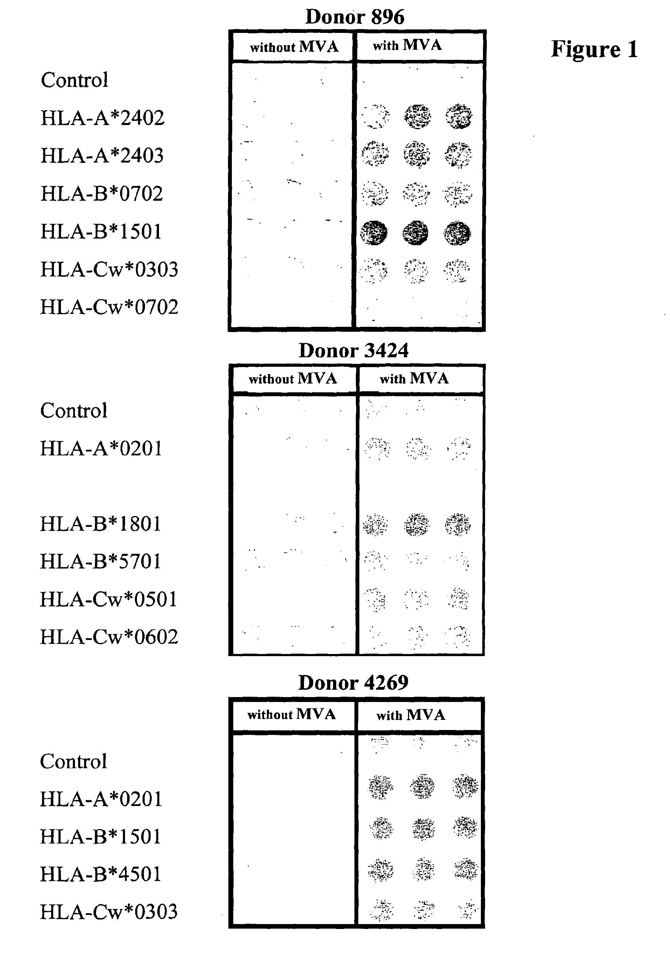

**FIG. 1 presents HLA restrictions of MVA reactive T cell populations.** CD8 positive T cells stimulated with MVA infected non-mature (donor 896) and mature dendritic cells (donors 3424 and 4269) were used as effector populations (20.000 CD8 positive T cells/well). COS-7 cells transiently transfected with HLA and infected with MVA were used as target cells.

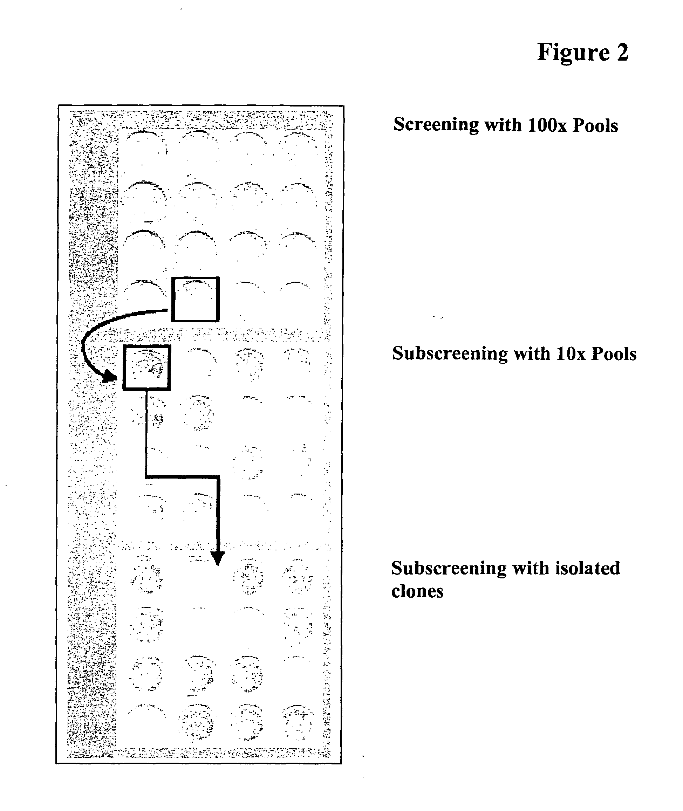

**FIG. 2 shows the identification of antigen A48R/B15.** COS cells were transfected with HLA-B*1501 and plasmid DNA (Vaccinia bank in vector pcDNA3.1His) and incubated for 20h at 37°C. After addition of effector cells incubation was for 20h at 37°C. Single positive clones were investigated for Vaccinia virus fragments by digesting with restriction enzymes and sequencing.

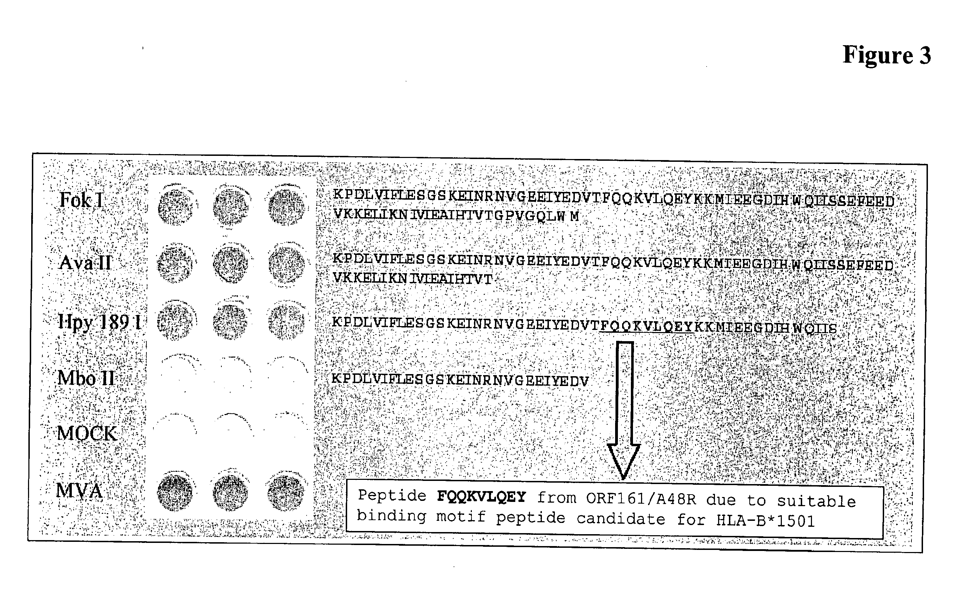

**FIG. 3 shows the identification of the peptide coding region of A48R cDNA.** A48R cDNA was digested using the enzymes indicated on the left hand side. The fragments were transcribed in vitro and subjected to polyadenylation. The thus produced mRNA fragments were transfected into K562/B* 1501 cells and screened for recognition by the MVA reactive 896 T cell population produced in Example 1 in an TFN-γ-ELISPOT assay. The amino acid sequences of the C-terminally truncated proteins encoded by the mRNA fragments are given on the right hand side.

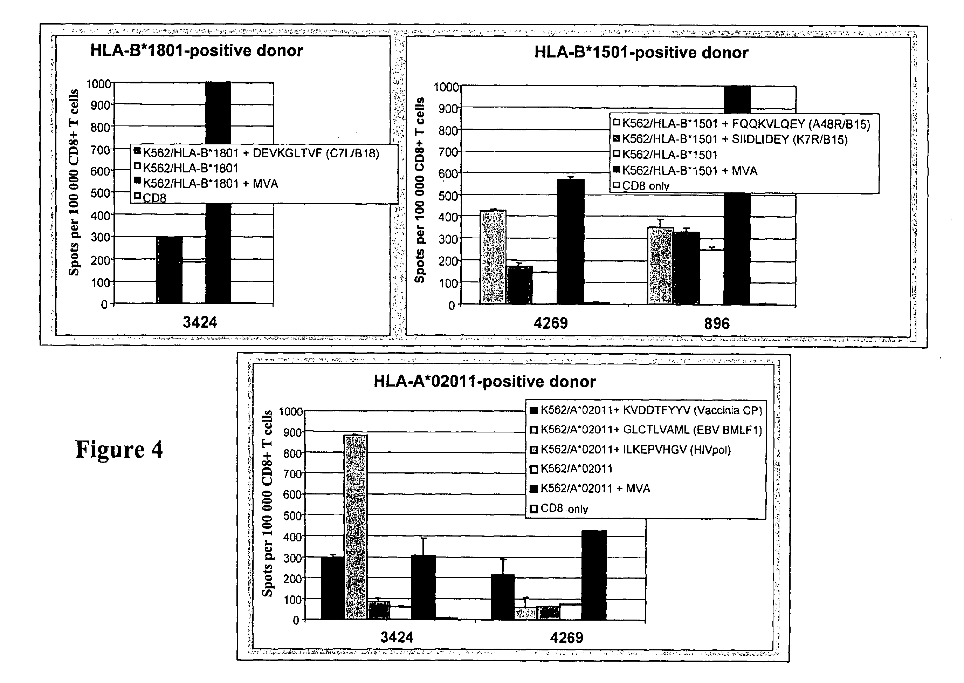

**FIG. 4 shows the testing of ex vivo PBMC.** Ex vivo PBMC of the donors indicated were used as effector populations. K562 cells stably transfected with the indicated HLA alleles were used as target cells.

### EXPERIMENTS

### EXPERIMENT 1: HLA RESTRICTIONS OF MVA REACTIVE T CELL POPULATIONS

CD8-positive T cells were purified from blood lymphocytes of three healthy donors (896, 3424, 4269) and were overall stimulated twice with MVA-BN-infected dendritic cells (DC) derived from the same individual (=autologous) in weekly intervals. In this way, T cell populations enriched for MVA-BN reactive T cells were produced. The lymphocyte populations were cryoconserved and were available for further testing (in the following designated as test populations).

In order to find out, which HLA restriction molecules were involved in the recognition of MVA-BN epitopes, the test populations were investigated in a 24h-IFN-γ-ELISPOT assay regarding the recognition of COS-7 cells which had simultaneously been transfected with HLA class I alleles of the donors and infected with MVA-BN (**Fig. 1**). In this way, a preferentially HLA-B*1501 restricted reactivity for donor 896 and a preferentially HLA-B*1801 (and additionally HLA-A*0201) restricted reactivity was shown for donor 3424. For donor 4269 apparently multiple restriction molecules were involved in the recognition of MVA-BN.

### EXPERIMENT 2: IDENTIFICATION OF PEPTIDE ANTIGENS

Plasmids containing different open reading frames (ORF) from the genome of MVA-BN (39 plasmids in total; provided by the company Bavarian Nordic) concomitantly with restricting HLA alleles were transfected into COS-7 cells. The transfectants were tested for recognition by the above described test populations. Only the test population of donor 896 recognized one of the plasmids in association with HLA-B*1501. It contained the MVA-BN ORF 028 (equivalent to Vaccinia CP ORF K7R).

The above mentioned test populations were further used to screen a Vaccinia bank in vector pcDNA3.1His (constructed by Dr. U. Sahin). The bank was divided into pools of 100 clones each. These were transfected into COS cells together with the respective HLA alleles of the respective lymphocyte donors and tested for recognition by the test population in IFN- γ-ELISPOT assays. Positive pools of hundreds were cloned stepwise. **Fig. 2** illustrates this procedure for the identification of the gene that codes for antigen A48R/B15. By this means, in addition to A48R/B15 also the gene for C7L/B18 was found.

For the identification of peptide coding gene regions, plasmids containing K7R, A48R and C7L cDNA, respectively, were linearized with enzymes whose restriction sites are in different ORF regions. Therefrom 3'-shortened mRNA fragments were generated *in vitro* which were transfected into HLA transfected K562 cells and tested for recognition by the above mentioned MVA-reactive T cell population. **Fig. 3** shows as an example the isolation of the peptide coding region of the MVA-BN ORF 161 (=Vaccinia CP ORF A48R). By means of a publicly available peptide prediction algorithm (http://www.syfpeithi.de/) peptides with lengths of nine and ten amino acids were identified and synthesized. These peptides were tested for recognition by the test populations of the three donors. The shortest positive peptides found are as follows:
**SEQ ID NO: 1: SIIDLIDEY:** presented by HLA-B*1501: antigen **K7R/B15;**
**SEQ ID NO: 2: FQQKVLQEY:** presented by HLA-B*1501: antigen **A48R/B15;**
**SEQ ID NO: 3: DEVKGLTVF:** presented by HLA-B*1801: antigen **C7L/B18**

The tests described so far were carried out using *in vitro* expanded T cell populations. In order to demonstrate that T cells against these epitopes are present in the peripheral blood in measurable frequencies unstimulated ("*ex vivo*") blood lymphocytes of donors 896 (positive for HLA-B*1501), 3424 (positive for HLA-A*02011 and HLA-B*1801) and 4269 (positive for HLA-B*1501) were investigated for recognition of the newly identified peptide antigens. As can be seen from **Fig. 4** approximately 0.3 to 0.4 % of CD8 positive T cells in the peripheral blood are directed against each of these peptides. As a positive control the reactivity against the known HLA-A2 presented peptide KVDDTFYYV (Terajima et al., 2003, J. Exp. Med. 197:927-932) was determined for the HLA-A*02011 positive donors in parallel.

## Claims

1. A peptide comprising an amino acid sequence as set forth in any one of SEQ ID NO: 1 to 3, or a variant thereof having substantially identical biological properties, with the proviso that the peptide or variant is not a naturally occurring virus protein.

2. The peptide or variant according to claim 1 which has an amino acid sequence as set forth in any one of SEQ ID NO: 1 to 3.

3. The peptide or variant according to claim 1 which is recognized by cytotoxic T lymphocytes and/or induces cytotoxic T lymphocytes.

4. The peptide or variant according to claim 3, wherein being recognized by cytotoxic T lymphocytes and/or inducing cytotoxic T lymphocytes is being recognized by cytotoxic T lymphocytes in an HLA-B-restricted manner and/or inducing cytotoxic T lymphocytes in an HLA-B-restricted manner.

5. The peptide or variant according to any one of claims 1 to 4 wherein the peptide or variant is capable of binding to HLA-B.

6. A nucleic acid encoding the peptide or variant according to any one of claims 1 to 5.

7. An expression vector comprising the nucleic acid according to claim 6.

8. A host cell transformed or transfected with the nucleic acid according to claim 6 or the expression vector according to claim 7.

9. An antibody, T cell or T cell receptor which specifically binds to a peptide or variant according to any one of claims 1 to 5 or to a complex of said peptide or variant with an HLA molecule, preferably HLA-B.

10. A pharmaceutical composition which comprises one or more components selected from the group consisting of (i) a peptide comprising an amino acid sequence as set forth in any one of SEQ ID NO: 1 to 3, or a variant thereof having substantially identical biological properties, (ii) a nucleic acid which codes for said peptide or variant, (iii) a host cell which expresses and/or presents said peptide or variant, (iv) an antibody which binds to said peptide or variant, (v) a T cell or T cell receptor which binds to said peptide or variant, (vi) isolated complexes between said peptide or variant and an HLA molecule, and optionally, a pharmaceutically acceptable carrier or adjuvant.

11. The pharmaceutical composition according to claim 10 which is a vaccine.

12. A kit for diagnosis of Orthopoxvirus infection in a biological sample and/or determining immune reactivity of a biological sample against Orthopoxvirus, which comprises a peptide comprising an amino acid sequence as set forth in any one of SEQ ID NO: 1 to 3, or a variant thereof having substantially identical biological properties.

13. The diagnostic kit according to claim 12 for determination of T cell immune reactivity against Orthopoxvirus in the biological sample.

14. The diagnostic kit according to claim 12 for determination of total antibodies, antibody IgG and/or antibodies IgM against Orthopoxvirus in the biological sample.

15. The diagnostic kit according to any one of claims 12 to 14 wherein said peptide or variant is pre-coated on the surface of a suitable support.

16. Use of a peptide comprising an amino acid sequence as set forth in any one of SEQ ID NO: 1 to 3, or a variant thereof having substantially identical biological properties, a nucleic acid encoding said peptide or variant, an expression vector comprising said nucleic acid, or any combination thereof, for the in-vivo- or in-vitro generation, activation and/or expansion of T cells, in particular CD8-positive cytotoxic T lymphocytes.

17. A method for inducing cytotoxic T lymphocytes comprising the steps of:
i) incubating an antigen-presenting cell with a peptide comprising an amino acid sequence as set forth in any one of SEQ ID NO: 1 to 3, or a variant thereof having substantially identical biological properties; or ii) expressing a peptide comprising an amino acid sequence as set forth in any one of SEQ ID NO: 1 to 3, or a variant thereof having substantially identical biological properties, in an antigen-presenting cell; and
iii) using the cell obtained in said step i) or said step ii) for stimulating a group of cells that contain a precursor cell of the cytotoxic T lymphocytes.

18. The method according to claim 17 wherein the cytotoxic T lymphocytes and the antigen presenting cells are allogeneic (allorestricted) with respect to the HLA molecule expressed by the antigen presenting cell.

19. The method according to claim 17 wherein the cytotoxic T lymphocytes and the antigen presenting cells are syngeneic (self-restricted) with respect to the HLA molecule expressed by the antigen presenting cell.

20. The method according to any one of claims 17 to 19 wherein the HLA molecule expressed by the antigen-presenting cell is HLA-B, more preferably HLA-B*1501 or HLA-B*1801.

21. A method for prophylaxis and/or treatment of Orthopoxvirus infection in a patient, which comprises administrating to the patient a prophylaxis and/or treatment effective amount of cytotoxic T lymphocytes obtainable by the method according to any one of claims 17 to 20.

22. A method of determining the T cell immune reactivity of a mammal against Orthopoxvirus comprising contacting a sample from said mammal with a peptide comprising an amino acid sequence as set forth in any one of SEQ ID NO: 1 to 3, or a variant thereof having substantially identical biological properties, and/or a complex between said peptide or variant and a HLA molecule and/or a complex between antigen presenting cells and said peptide or variant and determining the reactivity of T cells contained in said sample.

23. The method according to claim 22 for determining the protection in said mammal against Orthopoxvirus infection.

24. The method according to claim 23 wherein the protection is provided by vaccination.

25. A method for prophylaxis and/or treatment of Orthopoxvirus infection in a patient, which comprises the steps of (1) obtaining antigen presenting cells from said patient; (2) contacting said antigen presenting cells with a peptide comprising an amino acid sequence as set forth in any one of SEQ ID NO: 1 to 3, or a variant thereof having substantially identical biological properties, a nucleic acid encoding said peptide or variant, an expression vector comprising said nucleic acid, or any combination thereof ex vivo; and (3) reintroducing the antigen presenting cells into the patient.

26. A method for prophylaxis and/or treatment of Orthopoxvirus infection in a patient, which comprises administrating to the patient a prophylaxis and/or treatment effective amount of (i) a peptide comprising an amino acid sequence as set forth in any one of SEQ ID NO: 1 to 3, or a variant thereof having substantially identical biological properties, (ii) a nucleic acid which codes for said peptide or variant, (iii) a host cell which expresses and/or presents said peptide or variant, (iv) an antibody which binds to said peptide or variant, (v) a T cell or T cell receptor which binds to said peptide or variant, (vi) isolated complexes between said peptide or variant and an HLA molecule, or any combination thereof.

27. A method for diagnosis of Orthopoxvirus infection in a patient, comprising contacting a peptide comprising an amino acid sequence as set forth in any one of SEQ ID NO: 1 to 3, or a variant thereof having substantially identical biological properties, with a sample of a patient to be detected under conditions suitable for the interaction of antigen and antibody and/or antigen and T cells and detecting antibody and/or T cells bound to said peptide or variant.

28. The method according to claim 27 wherein said peptide or variant is coated on the surface of a suitable support.
